# EUROPÄISCHE PATENTSCHRIFT

(11) **EP 0 758 217 B1**
(45) Veröffentlichungstag und Bekanntmachung des Hinweises auf die Patenterteilung: **03.12.2003**
(21) Anmeldenummer: 95915758.7
(22) Anmeldetag: 18.04.1995
(51) Int. Cl.: A61F 2/36, A61B 17/17, A61F 13/15, A61L 2/06, A61L 31/00, A61L 27/00

(54) **GELENKPROTHESE**
JOINT PROSTHESIS
PROTHESE ARTICULAIRE

(30) Priorität: 02.05.1994 DE 4415378; 28.10.1994 DE 4438620
(43) Veröffentlichungstag der Anmeldung: 19.02.1997
(73) Patentinhaber: Elhami, Laghaollah, 81737 München (DE)
(72) Erfinder: Elhami, Laghaollah, 81737 München (DE)
(74) Vertreter: Schwan, Gerhard, Dipl.-Ing.
(86) Internationale Anmeldenummer: DE9500481
(87) Internationale Veröffentlichungsnummer: WO95029650

(56) Entgegenhaltungen:
- EP-A- 0 361 641
- EP-A- 0 636 346
- WO-A-86/04798
- WO-A-93/11713
- WO-A-93/22982
- CH-A- 202 657
- CH-A- 626 249
- DE-A- 2 808 740
- DE-A- 3 006 179
- DE-A- 3 516 743
- DE-A- 3 538 346
- DE-A- 3 840 471
- FR-A- 2 484 242
- FR-A- 2 532 543
- FR-A- 2 578 739
- FR-A- 2 686 503
- GB-A- 719 308
- GB-A- 1 448 111
- US-A- 2 397 545

## Beschreibung

Die Erfindung betrifft eine Gelenkprothese, für den mit einer Pfanne zusammenwirkenden Gelenkkopf eines Gelenkes, insbesondere eines Hüftgelenkes, gemäß Anspruch 1, sowie eine gelenkprothese für den mit einer Pfanne zusammenwirkenden Femurkopf eines Hüftgelenks gemäß Anspruch 38.

Eine Gelenkprothese mit den Merkmalen der Oberbegriffe der Patentansprüche 1 und 38 ist aus DE 35 38 346 A1 bekannt. Bei der bekannten Prothese ist die Kappe auf ihrer von der Pfanne abgewandten Unterseite von einer Basis abgeschlossen, mit welcher die Kappe auf einer Auflagefläche aufliegt, die zu diesem Zweck an dem Hüftkopf angefräst ist. Die Fixierung der Kappe auf dem Hüftkopf erfolgt mittels Knochenzement, der auf die angefräste Auflagefläche aufgetragen wird. Bei dem kappenseitigen Teleskopelement der bekannten Prothese handelt es sich um einen Bolzen, der frei verschiebbar in einer Hülse längsgeführt ist, die ihrerseits mit einem Halteteil in Form einer Laschenplatte fest verbunden ist. Der Halt der Kappe auf dem Knochensystem wird ausschließlich durch den Knochenzement bewirkt. Lockert sich diese Zementverbindung im Laufe der Zeit, verliert die Kappe ihren Halt.

Der Erfindung liegt demgegenüber die Aufgabe zugrunde, eine Gelenkprothese zu schaffen, die einfach, rasch und exakt implantiert werden kann, die, wenn überhaupt, nur eine vergleichsweise geringfügige Bearbeitung des Gelenkkopfes, z.B. den Abtrag von krankhaftem Knorpelgewebe, erfordert und die gegen Lockern auch im Langzeiteinsatz zuverlässig geschützt ist.

Diese Aufgabe wird mit den Merkmalen der Patentansprüche 1 und 38 auf überraschend einfache Weise gelöst.

Durch die Nutzung der Kappeninnenfläche als Anlagefläche für den Gelenkkopf kann in vielen Fällen auf das massive Anfräsen einer Auflagefläche verzichtet werden. In manchen Fällen, wenn der Kopf teilweise zerstört ist, kann der zerstörte Teil entfernt werden, wobei die Anlagefläche der Kappe der Form des so präparierten Kopfes angepaßt ist. Die erfindungsgemäß vorgesehene Hemmvorrichtung sorgt für eine sichere Verankerung der Kappe mit Bezug auf das Knochensystem, ohne daß es eines Aufzementierens der Kappe bedarf Insbesondere ist ein Abheben der Kappe von dem Gelenkkopf ausgeschlossen, während eine selbsttätige Verkürzung der Teleskopanordnung unter dem Einfluß von auf die Kappe einwirkenden Druckkräften beziehungsweise von zusätzlichen Zugkräften einer gegebenenfalls vorgesehenen Feder ohne weiteres möglich ist.

Der Begriff "Kappe" soll vorliegend auch ein Kappensegment umfassen .

Bei der aus der DE 35 38 346 A1 bekannten Gelenkprothese bilden die Kappe, die Teleskopanordnung und das Halteteil - bis auf die längsverstellbare Führung des Bolzens in der Hülse - ein statisches System, das eine Versteifung des Femurkopfes und des Oberschenkelhalses mit Bezug auf den Femurschaft bewirkt. Die bekannte Prothese schließt infolgedessen biomechanische Bewegungen des Femurkopfes und des Oberschenkelhalses gegenüber dem Femurschaft aus. Die Folge davon ist zwangsläufig ein nicht unbeträchtlicher Knochenabbau des Oberschenkelknochens im Bereich des Femurkopfes und des Oberschenkelhalses. Dem kann in weiterer Ausgestaltung der Erfindung durch die Merkmale des Anspruchs 2 wirkungsvoll begegnet werden. Die flexible und/oder gelenkige Ausbildung der Verbindung ermöglicht einen Ausgleich von Zug- und/oder Druckbewegungen des Gelenkkopfes und Halses. Biomechanische Bewegungen des Gelenkkopfes und Knochenhalses gegenüber dem Schaft werden mindestens teilweise zugelassen. Zug-, Druck- und Scherkräfte können somit z.B. über den Oberschenkelknochen weitergeleitet bzw. in diesen eingeleitet werden, wodurch eine Knochenerhaltung, ein Knochenaufbau und sogar eine Knochenneubildung erreichbar sind. Es empfiehlt sich, die Flexibilität und Gelenkigkeit der Prothese so zu wählen, daß die Flexibilität und Gelenkigkeit der Prothese nur derart bemessen bzw. begrenzt sind, daß eine eventuelle Bruchgefahr des Oberschenkelkopfes und Knochenhalses im Bereich der Prothese ausgeschlossen ist.

Vorteilhafte weitere Ausgestaltungen der Gelenkprothese ergeben sich aus den Unteransprüchen 3 bis 37 und 39 bis 54. So werden durch das Anlenken oder federelastische Abstützen des halteteilseitigen Teleskopelements an dem Halteteil bzw. der Befestigungsplatte (Ansprüche 8 bis 11) die natürlichen biomechanischen Bewegungen in dem Knochensystem, d.h. insbesondere die Bewegungen des Femurkopfes gegenüber dem Oberschenkelhals und des Oberschenkelhalses gegenüber dem Femurschaft. unterstützt. Außerdem gestaltet sich dadurch die Montage bzw. Festlegung der Prothese für den Operateur besonders einfach. Das Sperrelement gemäß den Ansprüchen 12 und 13 erlaubt es beispielsweise, bei Befund einer Fraktur im Oberschenkel nahe dem Femurkopf diesen Bereich zunächst für einige Zeit ruhigzustellen.

Ein Biegeelement kann optional grundsätzlich im Bereich des kappenseitigen und/oder des halteteilseitigen Teleskopelements vorgesehen sein. Durch das Anordnen des Biegeelements entsprechend Anspruch 15 wird zum einen sichergestellt, daß die Kappe im Bereich des Femurkopfes keinen Kippkräften und damit möglichen Momenten ausgesetzt ist, die nach kürzester Zeit eine Relativbewegung zwischen der Kappe und dem Femurkopf zur Folge haben könnten. Zum anderen aber ist gewährleistet, daß die Kappe und ihre Verankerungsvorrichtung kein gegenüber Zug- und/oder Druckbewegungen innerhalb des Hüftkopfes unempfindliches statisches System, sondern ein dynamisches System bilden, das auf solche Zugund/oder Druckbewegungen innerhalb des Oberschenkelknochens reagieren kann. Die verdrehsichere Ausbildung des Biegeelements (Ansprüche 16 und 17) wirkt einer Lockerung und auch einer Relativbewegung der Kappe gegenüber dem Femurkopf entgegen. Eine zwei- oder mehrteilige Ausbildung mindestens eines der Teleskopelemente (Anspruch 19) erlaubt es, die Prothese ohne Schwierigkeit, auch noch während des operativen Eingriffs, an örtliche Gegebenenheiten oder spezielle Beschaffenheiten individuell und zugleich besonders vielseitig anzupassen. Außerdem ist die Herstellung besonders einfach möglich.

Eine fakultative Drehsicherung gemäß Anspruch 34 wirkt zusätzlich einer möglichen Rotation der Kappe gegenüber dem Femurkopf entgegen. Zur Drehsicherung können unter anderem ein oder mehrere spitze Vorsprünge, Dorne oder dergleichen an der Innenfläche der Kappe vorgesehen sein. Es ist aber auch möglich, für eine Drehsicherung durch eine exzentrische Anordnung des kappenseitigen Teleskopelements (Anspruch 37) oder Stiels zu sorgen. Das Merkmal des Anspruchs 48 wirkt Schmerzen entgegen, die möglicherweise bei bestimmten Bewegungen infolge von Kippkräften auftreten. Eine Ausbildung der Kappe gemäß Anspruch 50 schafft die Möglichkeit, die Oberfläche des Femurkopfes mit Gelenkflüssigkeit in Kontakt gelangen zu lassen. Gleichzeitig ist ein Abrieb zwischen den Außenflächen von Kappe und Pfanne herabgesetzt. Eine gemäß Anspruch 51 aufgebaute Kappe erlaubt es, die Kappe an die jeweilige Größe und/oder Form des Hüftkopfs genau anzupassen, um eine nahezu spielfreie Anlage der Kappe an dem Femurkopf zu erhalten. Durch die Kappenausgestaltung gemäß Anspruch 54 läßt sich die Reibung zwischen Hüftkopf und Pfanne vermindern. Die Kappe mit abgeflachtem Pol (Anspruch 55) ist für einen eventuellen Einsatz der Prothese ohne künstliche Pfanne vorgesehen, wodurch eine eventuelle Protrusio Acetabuli vermieden wird.

Bevorzugte Ausführungsbeispiele der Erfindung sind nachstehend unter Bezugnahme auf die Zeichnungen näher erläutert. Dabei zeigen:
- Fig. 1: eine perspektivische auseinandergezogene Seitenansicht einer erfindungsgemäß ausgebildeten Prothese;
- Fign. 2 bis 5: Ansichten ähnlich Fig. 2 für abgewandelte Ausführungsformen der Prothese nach der Erfindung;
- Fig. 6: eine perspektivische Darstellung eines Steckelements mit daran angebrachter Kappe;
- Fig. 7: einen Schnitt durch eine Kappe mit exzentrisch angeordnetem kappenseitigem Teleskopelement;
- Fig. 8: eine perspektivische Darstellung einer abgewandelten Ausführungsform der Rastvorrichtung der Teleskopanordnung;
- Fig. 9: teilweise im Schnitt und teilweise in perspektivischer Darstellung ein Ausführungsbeispiel eines zweiteiligen Steckelements;
- Fig. 10: eine perspektivische, auseinandergezogene Ansicht einer weiteren Ausführungsform der erfindunggemäßen Prothese;
- Fign. 11 bis 13: perspektivische Darstellungen von verschiedenen Kappenausbildungen;
- Fig. 14: eine perspektivische Darstellung eines Kappeneinsatzes;
- Fig. 15: im Schnitt eine zweiteilig ausgebildete Kappe;
- Fig. 16: eine teils geschnittene, teils perspektivische Ansicht einer vereinfachten Ausführungsform der Prothese;
- Fign. 17: und 18a perspektivische Darstellungen von zwei weiteren abgewandelten Ausführungsformen der Prothese nach der Erfindung;
- Fig. 18b: eine perspektivische Teildarstellung der Teleskopanordnung, bei welcher die Teleskopelemente über ein Kerbverzahnung in gegenseitigem Eingriff stehen;
- Fig. 19: eine perspektivische, teilweise aufgebrochene Ansicht einer chirurgischen Vorrichtung zur Einbringung einer Bohrung;
- Fig. 20: eine auseinandergezogene Schnittdarstellung eines Teils der Vorrichtung nach Fig. 19 in Verbindung mit zwei unterschiedlichen Ausführungsformen des Bohrers;
- Fig. 21: eine perspektivische, auseinandergezogene Ansicht von Teilen der Vorrichtung nach den Fign. 19 und 20;
- Fig. 22: einen Querschnitt entlang der Linie XXII-XXII der Fig. 21;
- Fig. 23: eine Teildarstellung der Vorrichtung mit einer abgewandelten Ausführungsform des Rohrstutzens;
- Fign. 24 bis 27: abgewandelte Ausführungsformen der Aufnahmeeinrichtung;
- Fig. 28: eine weiter abgewandelte Ausgestaltung der Aufnahmeeinrichtung in Verbindung mit einer Justiervorrichtung und einem Einsatz für das Aufnahmeelement;
- Fig. 29: eine Teilansicht einer abgewandelten Ausführungsform der Vorrichtung;
- Fig. 30: eine Draufsicht auf den die Aufnahmeeinrichtung tragenden Teil gemäß Fig. 29;
- Fig. 31: eine Ansicht ähnlich Fig. 29 für eine weiter abgewandelte Ausführungsform der Vorrichtung;
- Fig. 32: eine Vorrichtung ähnlich den Fign. 19 und 20, bei welcher an Stelle einer Aufnahmeeinrichtung ein Fühler vorgesehen ist;
- Fign. 33 und 34: eine Teilansicht der Vorrichtung mit abgewandelter Ausführungsform des Rohrstutzens sowie eine in den Rohrstutzen gemäß Fig. 33 einschraubbare Aufnahmebuchse;
- Fig. 35: eine abgewandelte Ausführungsform der Aufnahmebuchse, die zur Aufnahme eines Kirschnerdrahtes bestimmt ist;
- Fig. 36: eine perspektivische Darstellung eines in Verbindung mit dem Kirschnerdraht nach Fig. 35 verwendbaren Bohrers;
- Fig. 37: eine perspektivische Ansicht eines meißel- oder hobelförmigen Instruments;
- Fig. 38: eine auseinandergezogene, teils geschnittene Ansicht einer weiter abgewandelten Ausführungsform der Vorrichtung;
- Fig. 39: eine auseinandergezogene Schnittdarstellung von Teilen einer weiteren Ausführungsform der Vorrichtung;
- Fig. 40: eine auseinandergezogene, teils perspektivische, teils geschnittene Darstellung einer vereinfachten Ausführungsform der Vorrichtung;
- Fig. 41: eine perspektivische Ansicht einer Ausführungsform der Vorrichtung zum Einbringen der Bohrung von der Gelenkkopfseite aus;
- Fig.42: eine perspektivische Darstellung einer weiteren Ausführungsform der erfindungsgemäßen Prothese;
- Fign.43a und 43b: perspektivische Teildarstellungen von weiter abgewandelten Ausführungsformen der Prothese nach der Erfindung; sowie
- Fign. 44 und 45: schematische Teilansichten weiter abgewandelter Ausführungsbeispiele der Vorrichtung zum Einbringen von Bohrungen.

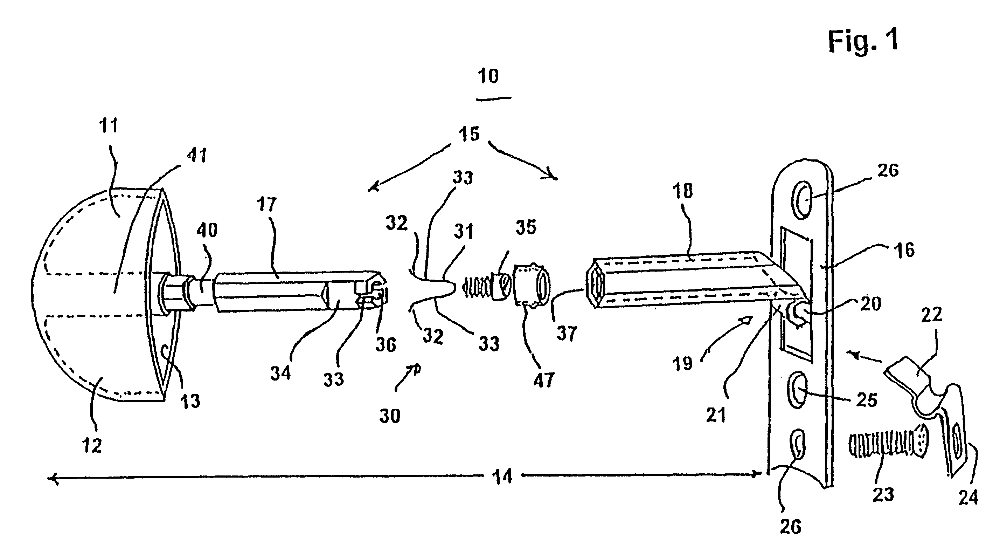

Die in Fig. 1 veranschaulichte Prothese 10 für einen Hüftgelenkkopf weist eine Kappe 11 auf, deren Außen- und Innenfläche 12 bzw. 13 jeweils mindestens näherungsweise kugelkappenförmig ausgebildet sind. Der Begriff "näherungsweise kugelkappenförmig" soll dabei sowohl weniger als halbkugelige Kappenformen als auch den in Fig. 1 und in anderen Figuren dargestellten Fall einschließen, daß der Umriß halbkugelig oder mehr als halbkugelig ist, oder daß sich an eine Halbkugelform ein insbesondere zylindrischer oder sich konisch erweiternder Abschnit anschließt. Im implanierten Zustand der Prothese 10 wirkt die Außenfläche 12 der Kappe 11 mit einer (nicht dargestellten) Pfanne zusammen, während die Kappeninnenfläche 13 auf dem präparierten Femurkopf bzw. dessen Knorpel drehgesichert wenigstens teilweise aufliegt. Dabei wird die Kappe 11 über eine Verankerungsvorrichtung 14 auf dem Femurkopf gehalten und mit Bezug auf den Femurschaft verankert. Zu der Verankerungsvorrichtung 14 gehören eine Teleskopanordnung 15 und ein Halteteil, das im veranschaulichten Ausführungsbeispiel als Befestigungsplatte 16 ausgebildet ist.

Die Teleskopanordnung 15 weist ein kappenseitiges Teleskopelement und ein halteseitiges Teleskopelement auf. Bei dem Ausführungsbeispiel der Fig. 1 ist das kappenseitige Teleskopelement ein Steckelement 17, während es sich bei dem halteteilseitigen Teleskopelement um ein Aufnahmeelement 18 handelt. Bei implantierter Prothese 10 stehen das Steckelement 17 und das Aufnahmeelement 18 in Teleskopeingriff miteinander, wobei die Länge der Teleskopanordnung 15 so bemessen ist, daß sie sich durch eine durchgehende Bohrung in dem Femurkopf, dem Oberschenkelhals und dem Femurschaft hindurcherstreckt. Das Aufnahmeelement 18 ist mit der Befestigungsplatte 16 über ein Drehgelenk 19 verbunden. Das Drehgelenk 19 weist eine von einem Quersteg 20 der Befestigungsplatte 16 bestimmte Drehachse auf, die im wesentlichen senkrecht zu der Längserstreckung der Teleskopanordnung 15 und im wesentlichen senkrecht zu der Längserstreckung der Befestigungsplatte 16 verläuft. Bei dem Ausführungsbeispiel gemäß Fig. 1 ist zur Bildung des Drehgelenks 19 das halteteilseitige Ende des Aufnahmeelements 18 mit einer den Quersteg 20 mindestens teilweise umgreifenden Gelenkbuchse 21 versehen. Das Aufnahmeelement 18 ist an der Befestigungsplatte 16 über eine Blattfeder 22 federelastisch abgestützt. Die Blattfeder 22 legt sich einerseits an die Außenfläche der Befestigungsplatte 16 und andererseits gegen die Unterseite des Aufnahmeelements 18 an, und sie wird mittels einer Schraube 23 gehalten, die durch Öffnungen 24 und 25 der Blattfeder 22 bzw. der Befestigungsplatte 16 hindurchgreift und in den Femurschaft eingeschraubt wird. Weitere Öffnungen in der Befestigungsplatte 16 zur Aufnahme von Befestigungsschrauben sind bei 26 angedeutet.

In die Verankerungsvorrichtung 14 ist eine richtungsabhängig wirkende Hemmvorrichtung integriert, bei welcher es sich im Falle des Ausführungsbeispiels der Fig. 1 um eine selbsttätig richtungsabhängig wirksam gemachte Kupplungseinrichtung in Form einer Rast- oder Schnappeinrichtung handelt. Insbesondere weist die Kupplungseinrichtung 30 eine V-förmig gebogene Feder 31 auf, die in zwei einander gegenüberliegenden, nach außen in entgegengesetzten Richtungen vorstehenden Rastvorsprüngen 32 endet. Die Feder 31 ist in einen Schlitz 33 am freien Ende des Steckelements 17 eingelegt. An den Schlitz 33 schließen sich in Richtung der Kappe 11 zu beiden Seiten des Steckelements 17 Abflachungen 34 an, gegen die sich die Feder 31 mit ihren Schenkeln 33 abstützen kann. Die Feder 31 ist in dem Schlitz 33 mittels einer Schraube 35 gesichert, die in eine Gewinde-Längsbohrung 36 des Steckelements 17 eingeschraubt wird. Die Rastvorsprünge 32 legen sich gegen einander diametral gegenüberliegende Anlageflächen 37 an der Innenseite des Aufnahmeelements 18 an. Diese Anlageflächen 37 sind mit einer Mehrzahl von Rastausnehmungen in Form eines sägezahnartigen Profils oder dergleichen versehen. Die Schraube 35 läßt sich auch bei zusammengebauter Prothese mittels eines Werkzeuges, insbesondere Schraubendrehers, lösen oder verstellen, das, bzw. der von dem halteteilseitigen Ende des Aufnahmeelements 18 durch dessen Innenraum hindurch eingeführt wird. Bei Anziehen der Schraube 35 wird Druck auf das halteteilseitige Ende der Feder 31 ausgeübt, und die Rastvorsprünge 32 werden radial nach außen vorgespannt. Wird auf die Kappe 11 eine Kraft in der von der Pfanne wegweisenden Richtung, das heißt in Richtung auf die Befestigungsplatte 16, ausgeübt, weichen die Rastvorsprünge 32 nach innen aus, so daß das Steckelement 17 sich weiter auf die Befestigungsplatte 16 zubewegen kann. Dadurch läßt sich bei der Implantation auf einfachste Weise diejenige Länge der Teleskopanordnung 15 einstellen, bei welcher die Kappeninnenfläche 13 an dem Gelenkkopf fest anliegt. Die Kupplungseinrichtung 30 sorgt aber auch für eine selbsttätige Längennachstellung der Teleskopanordnung 15, falls es bei dem betreffenden Patienten langfristig zu einem Schwund von Knochengewebe kommen sollte.

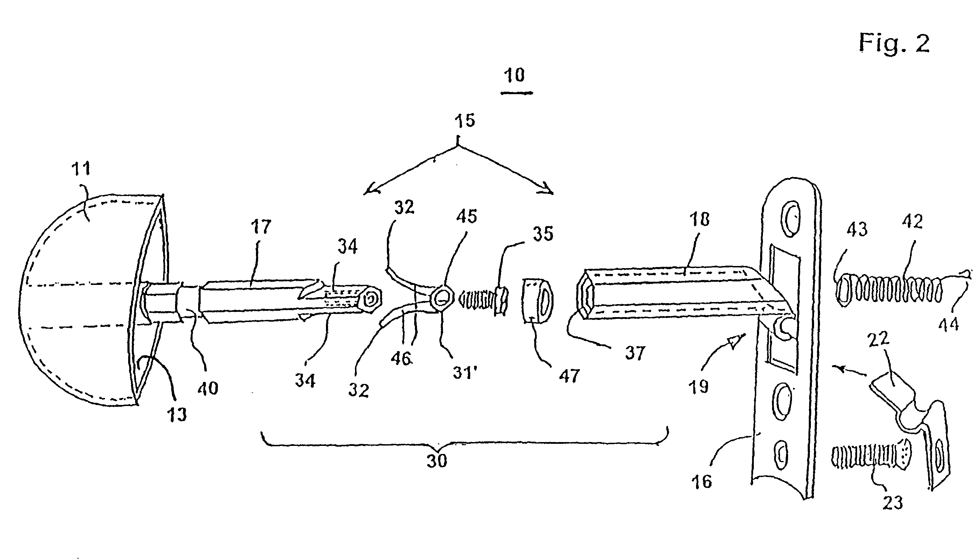

Wirkt dagegen auf die Prothese 10 eine Kraft ein, welche die Kappe 11 in der auf die Pfanne zuweisenden Richtung, d.h. weg von der Befestigungsplatte 16, zu verstellen sucht, stützen sich die Rastvorsprünge 32 in der betreffenden Rastausnehmung des Profils der Anlagefläche 37 ab. Eine ungewollte Vergrößerung der Längsabmessung der Teleskopanordnung 15 wird dadurch wirkungsvoll verhindert. Durch Lösen der Schraube 35 kann der Operateur jedoch die Kupplungseinrichtung 30 im Bedarfsfall ohne weiteres lösen und die Teleskopanordnung auseinanderziehen, wobei bei der Ausführungsform gemäß den Fign. 1 und 2 die Rastvorsprünge in dem Aufnahmeelement zurückbleiben und durch Druck in Richtung Halteplatte entfernt werden können.

Die Teleskopanordnung 15 ist ferner mit einem Biegeelement 40 versehen, das bei dem Ausführungsbeispiel gemäß Fig. 1 in das Steckelement 17 integriert ist. Das Biegeelement ist in einem Abstand von der Innenfläche 13 der Kappe 11 angeordnet, der dem etwa ein- bis dreifachen, insbesondere dem ungefähr eineinhalbfachen, des Innenradius der Kappe 11 entspricht. Das Biegeelement 40 läßt, gegebenenfalls im Zusammenwirken mit dem Drehgelenk 19, die natürlichen biomechanischen Bewegungen des Femurkopfes und des Halses mindestens teilweise zu. Anstelle des Biegeelements kann auch ein Gelenk vorgesehen sein. Das Biegeelement 40 oder ein entsprechendes Gelenk sind verdrehsicher, das heißt so ausgebildet, daß das Steckelement 17 in sich torsionssteif bleibt. Zusätzlich haben zweckmäßig das Steckelement 17 und das Aufnahmeelement 18 über mindestens einen Teil ihrer Längsabmessung einen profilierten, insbesondere eckigen oder elliptischen, Querschnitt. Im veranschaulichten Ausführungsbeispiel weist das Steckelement 17 in einem dem Aufnahmeelement 18 zugewendeten Bereich einen sechseckigen Außenumfang auf, während das Aufnahmeelement 18 mit einem komplementären sechseckigen Innenumfang versehen ist. Durch die drehfeste Verbindung von Steckelement 17 und Aufnahmeelement 18 und die verdrehsichere Ausbildung des Biegeelements 40 wird vermieden, daß sich die Kappe 11 nach dem operativen Einsetzen der Prothese 10 auf dem Femurkopf drehen und so bei häufiger Belastung lockern kann. Eine solche Lockerung würde dem Patienten Schmerzen bereiten und eine erneute Operation notwendig machen.

Das Biegeelement 40 oder ein entsprechendes Gelenk können unter anderem als torsionsfeste, insbesondere gehärtete, Feder, beispielsweise in Form einer Schraubenfeder oder einer Drillwelle ausgebildet sein. Ebenso ist eine Ausgestaltung des Biegeelements 40 oder des Gelenks als torsionsfestes Federgelenk oder als elastischer Einsatz denkbar. Ein entsprechender Einsatz kann beispielsweise als Kunststoffmanschette ausgebildet sein, die auf ihrem Außenumfang profiliert, z.B. mit einer Rändelung versehen, ist. Der Einsatz kann auch als Drahtgeflecht ausgeformt sein. Das Biegeelement 40 kann mit dem kappenseitigen Teleskopelement 17 aber auch einstückig verbunden sein, beispielsweise kann für diesen Zweck in das Steckelement 17 eine Spiralnut oder ähnliches eingebracht sein. Wenigstens im Bereich 41 der Kappe 11 weist das kappenseitige Teleskopelement 17 einen kreisrunden Querschnitt auf, um eine gegenüber kantigen Ausführungen mögliche Schmerzquelle ganz oder mindestens weitgehend auszuräumen. Bei dem Ausführungsbeispiel gemäß Fig. 1 erstreckt sich das kappenseitige Teleskopelement 17 im wesentlichen zentrisch von der Innenfläche 13 der Kappe 11 weg.

Bei der abgewandelten Ausführungsform der Prothese gemäß Fig. 2 ist zusätzlich eine Zugfeder 42 vorgesehen, welche das kappenseitige Teleskopelement 17 in der von der Pfanne wegweisenden Richtung, das heißt in Richtung auf die Befestigungsplatte 16, vorspannt. Die Zugfeder 42 sitzt innerhalb des hülsenförmigen Aufnahmeelements 18, und ihr kappenseitiges Ende 43 ist mit dem kappenseitigen Teleskopelement 17 über die Schraube 35 verbunden. Das andere Ende der Zugfeder 42 steht mit dem halteteilseitigen Teleskopelement 18 und/oder der Befestigungsplatte 16 in Verbindung. Beispielsweise ist das halteteilseitige Ende 44 der Feder 42 an dem äußeren Rand des Aufnahmeelements angehängt. Stattdessen könnte die Feder auch in den Quersteg 20 eingehängt sein. Anstelle der aus Draht gebogenen Feder 31 ist eine Feder 31' vorgesehen, die aus blattförmigem Federwerkstoff gefertigt ist. Die Feder 31' weist eine ringförmige Basis 45 auf, von der Federarme 46 abgebogen sind, welche mit ihren freien Enden die Rastvorsprünge 32 bilden.

Um während der Implantation ein unerwünschtes Vorragen der Rastvorsprünge 32 zu verhindern, bis die Rastvorsprünge 32 mit den zugehörigen Anlageflächen 37 in Eingriff kommen, kann ein Ring 47 vorgesehen sein. Der Ring 47 ist auf das Steckelement 17 aufgeschoben und hält zunächst die Rastvorsprünge 32 zurück. Beim Eintauchen des Steckelements 17 in das Aufnahmeelement 18 stößt der Ring 47 am freien Ende des Aufnahmeelements 18 an, und er wird dabei entlang dem Steckelement 17 in Richtung auf die Kappe 11 unter Freigabe der Rastvorsprünge 32 verschoben.

Im übrigen ist bei der Ausführungsform gemäß Fig.2 eine Kappe vorgesehen, deren Rand schiefwinklig zu der Kappenlängsachse verläuft, während in Fig.1 eine Kappe dargestellt ist, deren Rand in einer zu der Mittellängsachse der Kappe senkrechten Ebene verläuft. Es versteht sich, daß bedarfsweise mit der einen oder der anderen Kappenform gearbeitet werden kann.

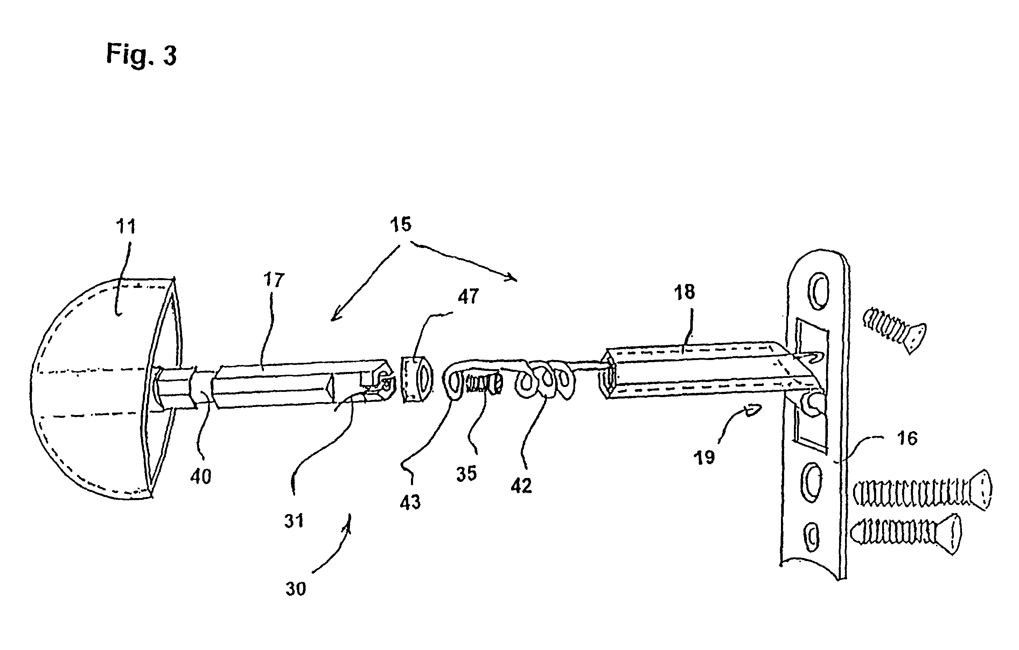

In Fig. 3 ist eine Ausführungsform der Prothese veranschaulicht, die weitgehend derjenigen nach Fig. 2 entspricht, wobei jedoch die fakultativ vorgesehene Blattfeder 22 weggelassen ist und die Feder 42 gemäß Fig. 2 in Funktionsstellung dargestellt ist.

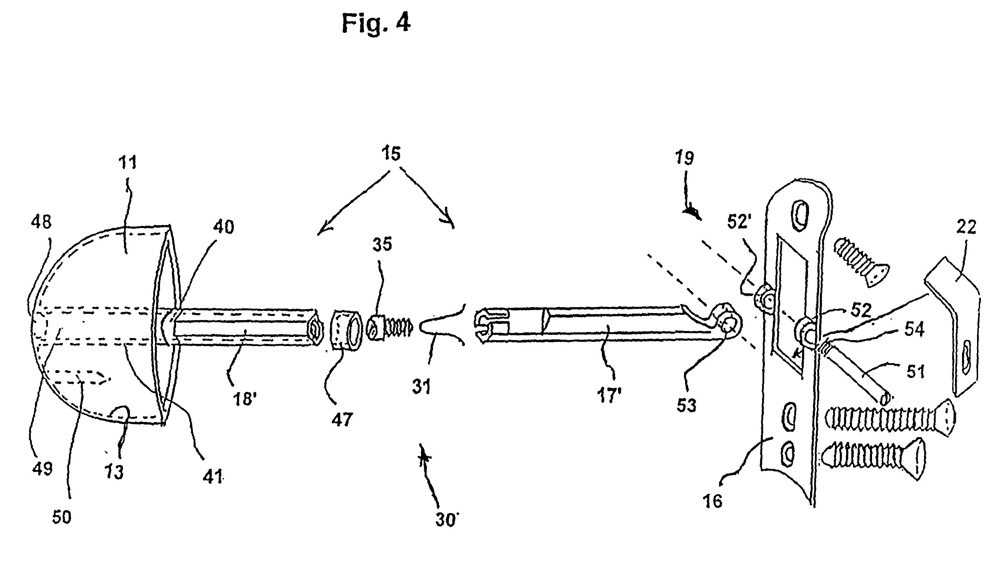

Das in Fig. 4 dargestellte Ausführungsbeispiel der Prothese unterscheidet sich von den zuvor beschriebenen Ausführungsbeispielen insbesondere dadurch, daß das halteteilseitige Teleskopelement als Steckelement 17' ausgebildet ist, während das zugehörige Aufnahmeelement 18' mit der Kappe 11 verbunden ist. Entsprechend den zuvor erläuterten Ausführungsbeispielen weist das kappenseitige Teleskopelement 18' wenigstens im Kappenbereich 41 einen kreisrunden Querschnitt auf. Auch hier ist das Biegeelement 40 in einem Abstand von der Innenfläche 13 der Kappe angeordnet, der dem etwa ein- bis dreifachen des Kappeninnenradius entspricht. Um die Schraube 35 bedarfsweise anziehen und lösen zu können, ist die Kappe 11 mit einer Durchgangsbohrung 48 versehen, die mit dem Längskanal 49 des Aufnahmeelements 18' ausgerichtet ist. Die Kappe 11 trägt bei diesem Ausführungsbeispiel ein Drehsicherungselement 50, das, wie veranschaulicht, als wenigstens ein spitzer Vorsprung, Dorn oder dergleichen ausgebildet sein kann, der von der Innenfläche 13 der Kappe 11 ausgehend etwa parallel und exzentrisch zu der Teleskopanordnung verläuft. Es versteht sich, daß das Drehsicherungselement 50 unterschiedlichste Abmessungen und Formen aufweisen kann.

Das Drehgelenk 19 weist bei diesem Ausführungsbeispiel einen Gelenkbolzen 51 auf, der in zwei seitlichen Ösen 52, 52' der Befestigungsplatte 16 gehalten ist und eine Gelenkbohrung 53 am halteteilseitigen Ende des Steckelements 17' durchgreift. Der Gelenkbolzen 51 trägt an seinem einen Ende ein Außengewinde 54, das mit einem entsprechenden Innengewinde der einen Öse 52' zusammenwirkt.

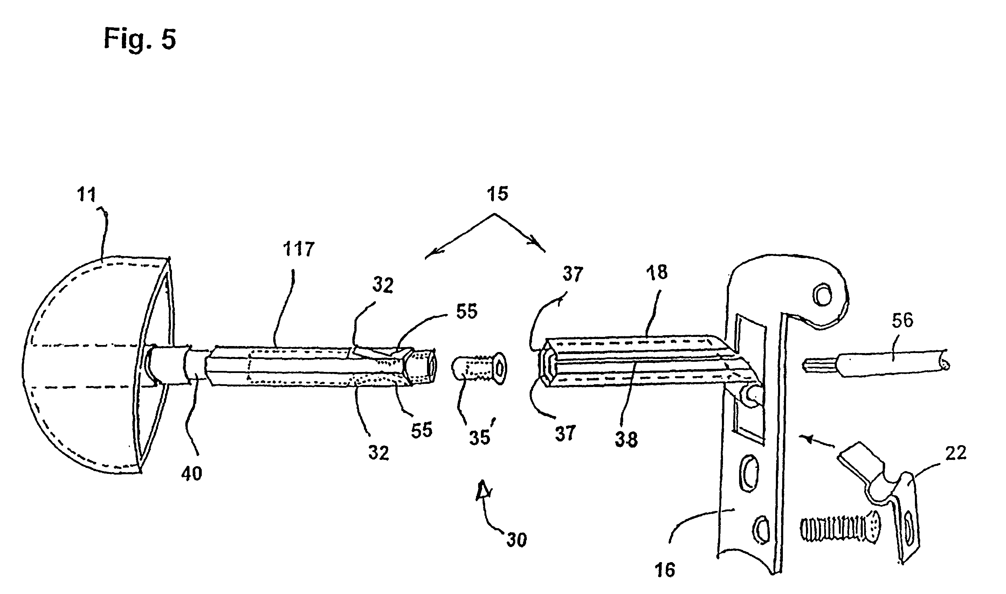

Fig. 5 zeigt ein weiter abgewandeltes Ausführungsbeispiel der Prothese, bei dem die Rastvorsprünge 32 am freien Ende von Federarmen 55 vorgesehen sind, die an einem Steckelement 117 angebracht, beispielsweise mit diesem verschweißt oder verlötet sind oder die mit einem Teil des Steckelements 117 in einstückiger Verbindung stehen, beispielsweise aus diesem herausgestanzt und -gebogen sind. Entsprechend den zuvor erläuterten Ausfürhungsbeispielen wird die - in diesem Fall als Imbusschraube dargestellte - Schraube 35' in ein Innengewinde an dem dem Aufnahmeelement 18 zugewendeten Ende des Steckelements 117 eingeschraubt, um die Federarme 55 nach außen vorzuspannen. Die Schraube 35' läßt sich mittels eines Werkzeuges 56 verstellen, das durch den Längskanal des Aufnahmeelements 18 hindurchgesteckt werden kann. Im implantierten Zustand nimmt die Schraube 35' eine Stellung ein, in welcher sich die Rastvorsprünge 32 in Ausnehmungen einlegen, die von den vorzugsweise sägezahnartig ausgebildeten Anlageflächen 37 des Aufnahmeelements gebildet werden. Erweist es sich als notwendig, die Teleskopanordnung auseinanderzuziehen, wird die Schraube 35' herausgedreht, und die Federarme 55 kehren in eine Ausgangsstellung zurück, in der sie außer Eingriff mit den Anlageflächen 37 sind. Bei dieser Ausführungsform ist das Aufnahmeelement 18 mit einem Längsschlitz 38 versehen, der es erlaubt, für eine im wesentlichen spielfreie Anpassung der Querschnittsinnenabmessungen des Aufnahmeelements an die Querschnittsaußenabmessungen des Steckelements 117 zu sorgen.

Es versteht sich, daß die Hemmvorrichtung im Rahmen der vorliegend beanspruchten Erfindung in vielgestaltiger Weise abgewandelt werden kann.

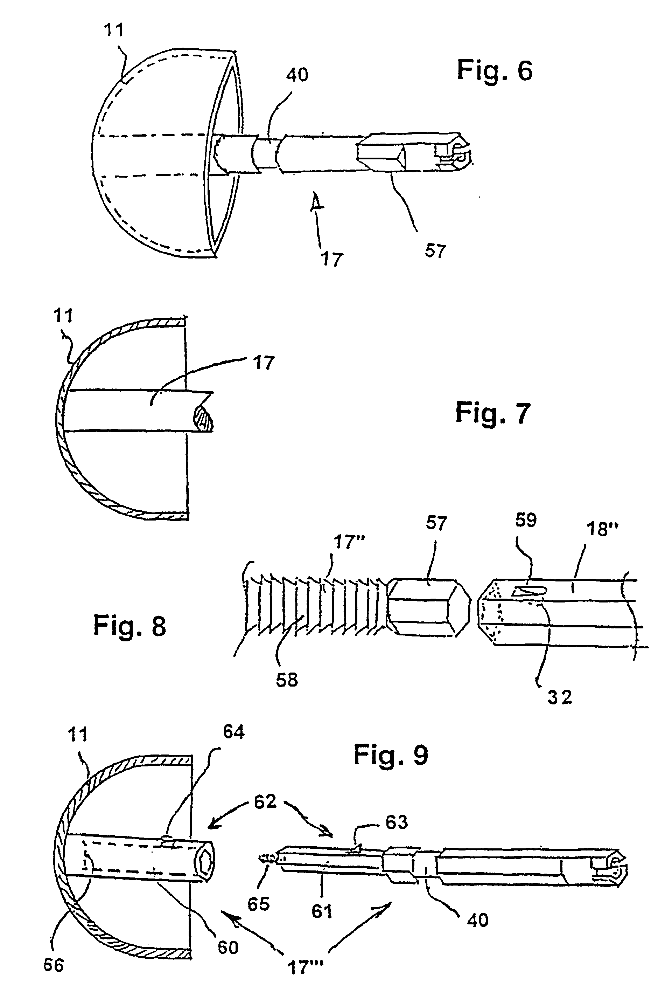

Entsprechend Fig. 6 kann der Teil 57 des Steckelements 17, der einen profilierten, im gezeigten Ausführungsbeispiel sechseckigen, Querschnitt aufweist, relativ kurz sein und das Steckelement 17 im übrigen mit kreisrundem Querschnitt ausgestaltet sein. Wesentlich ist nur, daß der Teil 57 mit unrundem Querschnitt von ausreichender Länge ist, um im Zusammenwirken mit dem komplementären Aufnahmeelement eine gegenseitige Drehung der die Teleskopanordnung bildenden Elemente zu verhindern.

Fig. 7 zeigt ein Ausführungsbeispiel, bei welchem das kappenseitige Teleskopelement, z.B. das Steckelement 17, sich in einem vorbestimmten Abstand von der Mittelachse der Kappe - übereinstimmend mit dem Exzentrizitätsgrad der weiter unten beschriebenen Bohrvorrichtung - parallel zu dieser Achse erstreckt. Eine solche Anordnung kann allein oder zusätzlich zu den erläuterten Maßnahmen für eine Drehsicherung sorgen, die ein Drehen der Kappe 11 gegenüber dem Gelenkkopf verhindert.

Fig. 8 zeigt ein Ausführungsbeispiel der Teleskopanordnung, bei welchem an einen profilierten, beispielsweise sechseckigen Teil 57 des Steckelements 17" ein Abschnitt 58 anschließt, der ein sägezahnartig ausgebildets Außenprofil hat. Aus der Wand des Aufnahmeelements 18" ist eine Federzunge 59 ausgestanzt und nach innen abgebogen. Dabei bildet das freie Ende der Federzunge 59 den Rastvorsprung 32, der im zusammengebauten Zustand mit einer der Rastausnehmungen des Abschnitts 58 in Sperreingriff kommt.

Gegebenenfalls können das Steckelement und/oder das Aufnahmeelement der Teleskopanordnung auch ihrerseits aus zwei oder mehr in Längsrichtung der Teleskopanordnung hintereinander liegenden Teilen bestehen, die ineinander drehfest einsteckbar oder einschraubbar und gegen selbsttätiges Lösen gesichert sind.

Fig. 9 zeigt als Beispiel ein zweiteiliges Steckelement 17''' aus zwei Teleskopteilen 60 und 61. Die beiden Teleskopteile 60, 61 sind mit komplementären profilierten, im veranschaulichten Ausführungsbeispiel sechseckförmigen Querschnitten versehen, die beim Zusammenstecken der Teleskopteile 60 und 61 für eine drehfeste Verbindung sorgen. Die beiden Teile 60, 61 des Steckelements 17''' sind mittels einer Rast- oder Schnappeinrichtung 62 zueinander festlegbar. Diese Einrichtung kann in vielfältiger Weise aufgebaut sein, z.B. in der gleichen Weise wie die Kupplungseinrichtung 30 zum gegenseitigen Verbinden von Steckelement 17 und Aufnahmeelement 18. Beim Ausführungsbeispiel der Fig. 9 sind eine oder mehrere, zweckmäßig sägezahnartige Rastvorsprünge oder -nasen 63 vorgesehen, der bzw. die sich von dem Teleskopteil 61 radial nach außen erstrecken, um in eine oder mehrere entsprechende Ausnehmungen 64 des Teleskopteils 60 einzugreifen. Eine an dem Teleskopteil 61 stimseitig befestigte und in Längsrichtung des Steckelements 17 wirkende Feder 65 stützt sich am Bohrgrund 66 des Teleskopteils 60 ab, um nach dem gegenseitigen Wirkeingriff der Rastvorsprünge bzw. -nasen 63 und Ausnehmungen 64 für eine spielfreie, sichere Halterung der beiden ineinander gesteckten Teile 60, 61 zu sorgen.

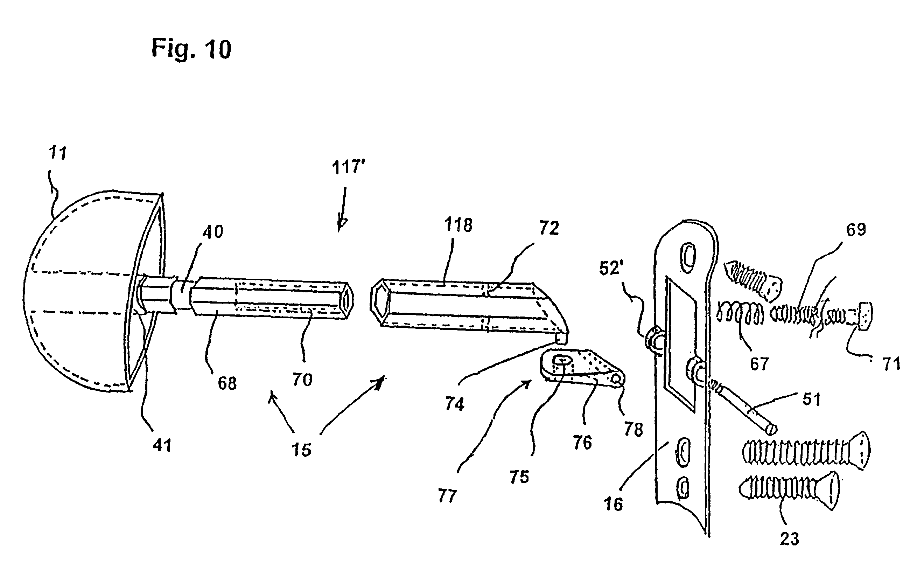

Bei der weiter abgewandelten Ausführungsform der Prothese gemäß Fig. 10 wird die richtungsabhängig wirkende Hemmvorrichtung, die eine Verstellung der Kappe 11 gegenüber dem Halteteil 16 in der auf die Befestigungsplatte 16 zuweisenden Richtung erlaubt und in der von der Befestigungsplatte 16 wegweisenden Richtung erschwert, von einer Federvorspanneinrichtung gebildet, zu der eine Vorspannfeder 67 gehört. Das Steckelement 117' weist bei dieser Ausführungsform ein mit der Kappe 11 verbundens Teil 68 und ein dazu im wesentlich gleichachsig angeordnetes Teil 69 auf, die miteinander lösbar verbunden sind. Im veranschaulichten Ausführungsbeispiel wird das Teil 69 von einer Schraube gebildet, die in eine Innengewindebohrung 70 des Teils 68 eingeschraubt ist. Das Teil 69 weist eine Außenschulter 71 auf, gegen welche sich die Vorspannfeder 67 mit ihrem in Fig. 10 rechten Ende abstützt. Das andere, in Fig. 10 linke Ende der als Druckfeder ausgebildeten Vorspannfeder 67 liegt im montierten Zustand an einer Innenschulter 72 des Aufnahmeelements 118 an. Die von der Vorspannfeder 67 ausgeübte Kraft zieht das Steckelement 117' zusammen mit der Kappe 11 in Richtung auf die Befestigungsplatte 16. Ebenso wie bei den zuvor beschriebenen Ausführungsformen paßt sich auf diese Weise die Teleskopanordnung in ihrer Länge selbsttätig den natürlichen Gegebenheiten am Implantationsort auch dann an, wenn es nach der Implantation zu Knochenresorption oder dergleichen kommt.

Das Aufnahmeelement 118 trägt an seinem der Befestigungsplatte 16 zugewendeten Ende einen Gelenkbolzen 74, der in eine Gelenkbohrung 75 einer Gelenkplatte 76 drehbar eingesetzt ist. Der Gelenkbolzen 74 und die Gelenkbohrung 75 bilden zusammen ein Drehgelenk 77, dessen Drehachse im wesentlichen senkrecht zu der Längserstreckung der Teleskopanordnung und im wesentlichen parallel zu der Längserstreckung der Befestigungsplatte 16 verläuft. Der Gelenkbolzen 51 erstreckt sich im zusammenmontierten Zustand durch eine weitere Gelenkbohrung 78 der Gelenkplatte 76, wobei die Achse der Gelenkbohrung 78 senkrecht zu der Längsachse der Teleskopanordnung steht.

Die bei den erläuterten Ausführungsbeispielen vorgesehene gelenkige Verbindung zwischen Teleskopanordnung und Befestigungsplatte stellt sicher, daß die Prothese nach der Operation in den Oberschenkelknochen dynamisch bleibt, so daß Zug-, Druck- und Scherkräfte auf nahezu natürliche Art von der Prothese weitergeleitet bzw. in den Femurkopf und -hals eingeleitet werden können. Die gelenkige Anbindung der Teleskopanordnung hat ferner den Vorteil einer wesentlich vereinfachten Handhabung der Prothese während des operativen Eingriffs, insbesondere bei deren Befestigung an der Oberfläche des lateralen Oberschenkelknochens. So ist eine flächige Anlage der Befestigungsplatte 16 an der Oberfläche des lateralen Oberschenkelknochens unabhängig von dem Winkel zwischen den Mittellängsachsen des Oberschenkelknochens und der die Teleskopanordnung aufnehmenden Bohrung möglich.

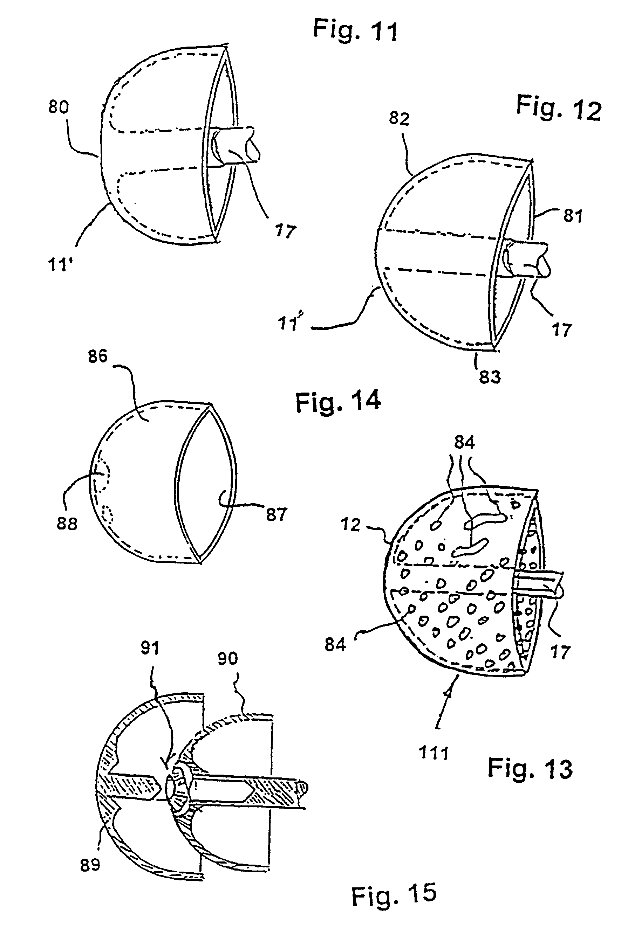

Die in Fig. 11 dargestellte Kappe 11' ist in ihrem Polbereich 80 abgeplattet. Außerdem kann die Kappe gegebenenfalls im Bereich des Kappenrandes nach außen verdickt sein. Durch solche Maßnahmen läßt sich je nach der Beschaffenheit der natürlichen Gelenkpfanne eine gegebenenfalls nicht zu einer Protrusio Acetabuli führende Kappenprothese ohne künstliche Pfanne implantieren.

Wie aus Fig. 12 folgt, liegt der Rand 81 der Kappe 11 mindestens zu einem größeren Teil in einer Ebene, die derart schiefwinklig zu der Längsachse der Prothese bzw. der Teleskopanordnung steht, daß der Gelenkkopf von der Kappe im implantierten Zustand mindestens näherungsweise gleichmäßig überdeckt wird. Dabei ist zu erkennen, daß sich an einen mindestens näherungsweise halbkugeligen Teil 82 der Kappe ein mehr oder weniger zylindrischer Kappenteil 83 anschließt.

Fig. 13 zeigt eine siebförmig ausgebildete Kappe 111, die mit einer Vielzahl von durchgehenden Ausnehmungen oder Bohrungen 84 versehen ist, um durch eine Verkleinerung der Kontaktflächen mechanischen Abrieb und Wärmeentwicklung zwischen Kappe und Pfanne zu reduzieren. Über die Bohrungen 84 erfolgt eine besonders wirkungsvolle Versorgung des Femurkopfes mit Gelenkflüssigkeit. Die Bohrungen 84 sind zur Verminderung eines Abriebs der Pfanne an der Kappenaußenfläche 12 entgratet oder trichterförmig ausgebildet.

Fig. 14 zeigt einen Einsatz 86, der als Innenteil der Kappe 11 gegebenenfalls in die Kappe einsetzbar ist und dessen Innenfläche 87 als Anlagefläche der Form des präparierten oder unpräparierten Gelenkkopfes mindestens näherungsweise angepaßt ist. Der Einsatz 86 wird aus einer Gruppe von Einsätzen unterschiedlicher Wandstärke und/oder unterschiedlicher Form zur Anpassung des Innendurchmessers und der Innenform der Kappe an den Durchmesser und die Form des jeweiligen Gelenkkopfs ausgewählt und eingesetzt. Auf diese Weise kann eine mindestens nahezu spielfreie Anlage der Kappe an dem Femurkopf sichergestellt werden. Der Einsatz 86 ist mit einer zentralen Öffnung 88 versehen, durch die sich im zusammengebauten Zustand das kappenseitige Teleskopelement hindurcherstreckt. Der Einsatz 86 kann beispielsweise aus Polyethylen gefertigt sein.

Entsprechend Fig. 15 kann die Kappe, z.B. die Kappe 11, zusätzlich zweiteilig ausgebildet sein, wobei ein schalenförmiges Oberteil 89 mit seiner Innenfläche auf der Außenfläche eines schalenförmigen Unterteils 90 aufliegt. Gegebenenfalls sind Oberteil 89 und Unterteil 90 über eine Lageranordnung 91 gegenseitig abgestützt. Die Lageranordnung 91 kann beispielsweise ein Nadellager in kegelförmiger Anstellung umfassen.

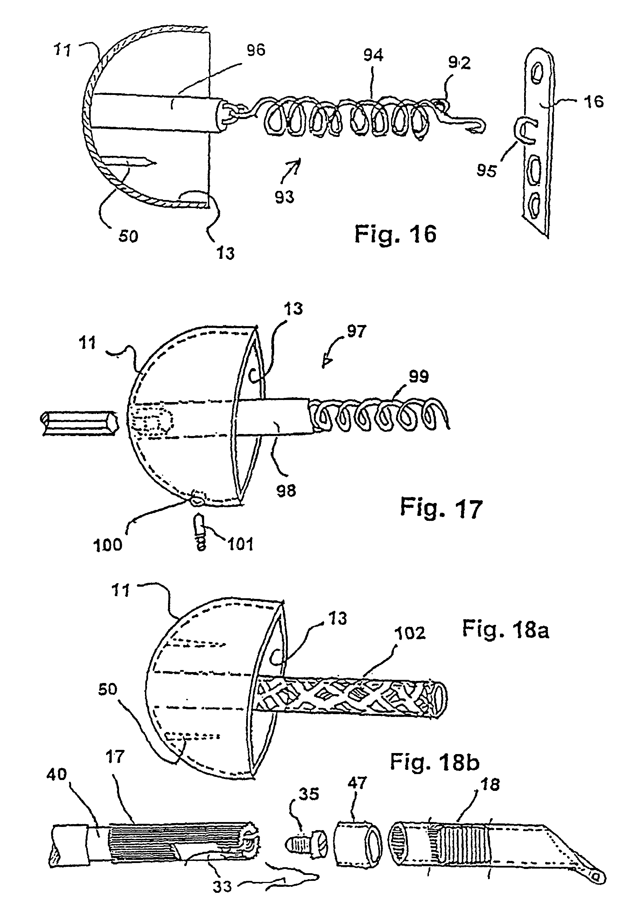

Die Prothese 10 gemäß Fig. 16 ist in vereinfachter Ausführung ohne Teleskopanordnung ausgebildet. Sie weist eine Hemmvorrichtung 93 auf, die eine gegenseitige Verstellung zwischen der Kappe 11 und der Befestigungsplatte 16 in der von der Befestigungsplatte 16 wegweisenden Richtung im Vergleich zu einer Verstellung in der auf die Befestigungsplatte 16 zuweisenden Richtung erschwert. Zu der Hemmvorrichtung 93 gehört mindestens ein federelastisches Zugelement 94, das mit seinem in Fig. 16 rechten Ende in eine Öse 95 der Befestigungsplatte 16 eingehängt wird. Das andere, in Fig. 16 linke Ende des Zugelements 94 steht mit einem Stiel 96 in Eingriff, der sich von der Innenfläche 13 der Kappe 11 weg erstreckt. Bei der Implantation kann das Zugelement mittels eines Werkzeuges an einer Öse 92 gefaßt und zum Einhängen in die Öse 95 gespannt werden. Zur Drehsicherung trägt die Kappe 13 mindestens ein Drehsicherungselement 50. Daneben oder zusätzlich kann bei dieser wie bei allen anderen Ausführungsformen der Stiel exzentrisch mit Bezug auf die Kappe angeordnet sein (Fig. 7).

Die Figuren 17 und 18 zeigen weiter abgewandelte Ausführungsformen der Gelenkprothese, denen gemeinsam ist, daß der von der Kappe 11 abstehende Stiel selbst als Verankerungselement ausgebildet ist. Bei der Ausführungsform gemäß Fig. 17 ist ein Stiel 97 vorgesehen, der einen mit der Kappe 11 verbundenen zylindrischen Abschnitt 98 und einen daran anschließenden, im wesentlichen korkenzieherförmigen Abschnitt 99 aufweist. Der Stiel 97 ist zentrisch mit Bezug auf die Kappe 11 angeordnet, und er ist mit seinem korkenzieherartigen Abschnitt 99 in die Bohrung und Knochengewebe einschraubbar, wobei der Stiel 97 in einer Bohrung geführt ist, die in den Gelenkkopf und daran anschließende Knochenteile eingebracht ist. Die Kappe 11 ist mit mindestens einer exzentrischen Ausnehmung 100 versehen, durch die hindurch ein Drehsicherungselement 101 von außen in den Gelenkkopf einbringbar ist. Das Drehsicherungselement 101 kann unter anderem als Dorn ausgebildet sein, das durch die Ausnehmung 100 hindurch eingeschlagen wird. Das Drehsicherungselement 101 kann aber auch mit einem Außengewindeabschnitt versehen sein, der mit einem Innengewinde der Ausnehmung 100 zusammenwirkt, um das Drehsicherungselement einzuschrauben. Vorzugsweise ist die Anordnung so getroffen, daß das Drehsicherungselement 101 in der Kappe 11 versenkt wird.

Bei der weiter abgewandelten Ausführungsform gemäß Fig. 18a ist ein Stiel 102 vorgesehen, der über mindestens einen Teil seiner Längsabmessung als Hülse mit gitterartiger Wand ausgebildet ist. In das Gitter kann nach der Implantation Knochengewebe einwachsen. Der Stiel 102 kann aber auch mindestens teilweise als Bolzen mit durchgehend poröser Struktur oder mindestens poröser Oberflächenstruktur ausgebildet sein. Während der Stiel 102 in Fig. 18a zentrisch mit Bezug auf die Kappe 11 dargestellt ist, versteht es sich, daß stattdessen zur Drehsicherung auch eine exzentrische Anordnung in Frage kommt.

Drehsicherungselemente 50 erstrecken sich ausgehend von der Innenfläche der Kappe parallel zu dem Stiel 102. Es versteht sich, daß statt dessen auch eines oder mehrere der Drehsicherungselemente 101 gemäß Fig. 17 verwendet werden können oder umgekehrt bei der Prothese nach Fig. 17 mindestens eines der Drehsicherungselemente 50 vorhanden ist.

Die verschiedenen Teile der Gelenkprothese können grundsätzlich aus beliebigem zweckentsprechendem Werkstoff gefertigt sein, insbesondere nichtrostendem Metall, wie Edelstahl, Titan, Chrom, oder dergleichen, und/oder Legierungen der vorstehend genannten Metalle und/oder Kunststoff, wie insbesondere Polyethylen, und/oder keramischen Werkstoffen, oder aus Kombinationen solcher Werkstoffe.

Die Anlageflächen für den oder die Rastvorsprünge können gegebenenfalls auch unprofiliert ausgebildet und/oder von einem Überzug gebildet sein, in den sich der Rastvorsprung einkrallen kann. Im Falle der Prothesen gemäß den Figuren 1 bis 16 kann zusätzlich eine nicht dargestellte Hülse vorgesehen sein, die in die Bohrung einsetzbar ist und die im implantierten Zustand die Teleskopanordnung bzw. das Zugelement und/oder den Stiel über mindestens einen Teil ihrer Längsabmessung umschließt. Eine solche Hülse aus Kunststoff kann einen Kontakt von Metallteilen der Prothese mit natürlichem Gewebe verhindern. Stattdessen oder zusätzlich können metallische Teile der Prothese auch mit einem nichtmetallischen Überzug, insbesondere Kunststoffüberzug, versehen sein. Eine starre Hülse, z. B. Metallhülse, kann benutzt werden, um im Bedarfsfall das Biegeelment 40 zu versteifen. Eine entsprechende Versteifung kann gegebenenfalls aber beispielsweise auch dadurch erreicht werden, daß ein Aufnahmeelement vorgesehen wird, das ausreichend lang ist, um sich über ein im Steckelement der Teleskopanordnung angeordnetes Biegeelement hinwegzuerstrecken. Bei den veranschaulichten Ausführungsbeispielen ist durch entsprechende Querschnittsform der Teleskopelemente für eine drehfeste gegenseitige Verbindung dieser Elemente gesorgt. Die Teleskopelemente können grundsätzlich aber auch einen kreisförmigen Querschnitt aufweisen, insbesondere dann, wenn auf geeignete andere Weise einem Verdrehen der Kappe auf dem Gelenkkopf vorgebeugt ist, z. B. durch exzentrische Anordnung des kappenseitigen Teleskopelements, durch Verwendung mindestens eines Drehsicherungsdorns, oder dergleichen .

Bei der Ausführungsform gemäß Fig. 18b ist das Steckelement mit einer Außenkerbverzahnung versehen, die mit einer kerbverzahnten Nabe am Anfang des Aufnahmeelementes 18 zusammenwirkt, um für eine Drehsicherung zwischen Steck- und Aufnahmeelement zu sorgen. An die kerbverzahnte Nabe schließt in Achsialrichtung eine Anlagefläche mit sägezahnartigem Profil an, wie sie weiter oben z. B. anhand der Fig. 1 näher erläutert ist.

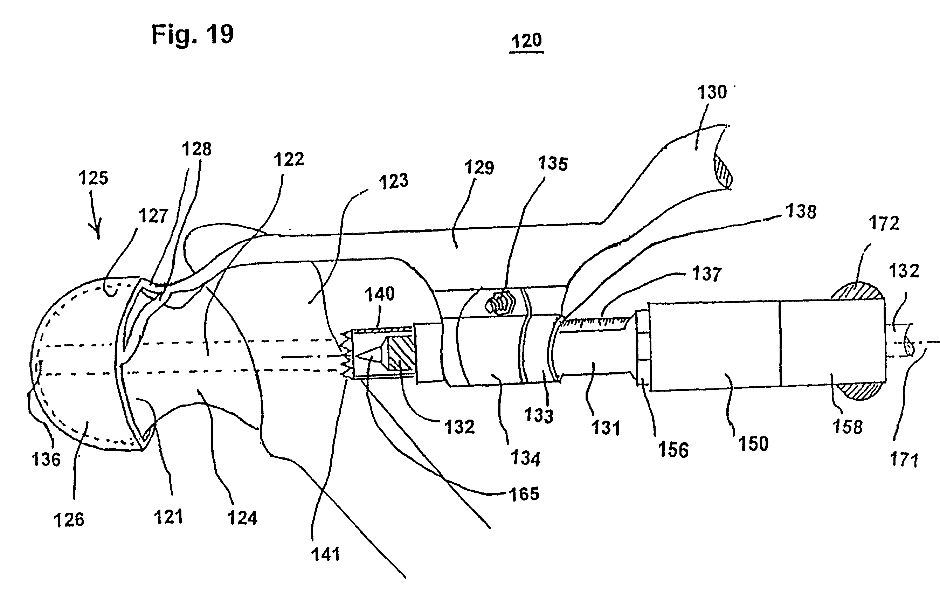

Fig. 19 zeigt ein Ausführungsbeispiel einer chirurgischen Vorrichtung 120 zum Einbringen einer genau vorher festgelegten, achsengerechten, zu dem bei 121 angedeuteten Femurkopf zentrischen Bohrung 122 von der Femurschaftseite aus, d.h. durch den Oberschenkelbeinoder Femurschaft 123, den Oberschenkelhals 124 und den Femurkopf 121. Die Vorrichtung 120 weist eine Aufnahmeeinrichtung 125 mit einem kugelkappenförmig ausgebildeten Aufnahmeelement 126 auf, das zur wenigstens teilweisen Aufnahme des Femurkopfes 121 ausgebildet ist. Dabei kann das Aufnahmeelement 126 in der bei Fig. 19 angedeuteten Weise über den Femurkopf 121 geschoben werden, und die Innenform des Aufnahmeelements 126 ist an die Außenform des Femurkopfes derart angepaßt, daß die Innenfläche 127 des Aufnahmeelements 126 mit wenigstens einem Teil der Außenfläche des Femurkopfes zur Anlage bringbar ist. Das Aufnahmeelement 126 ist über Haltestege 128 mit einem seitlichen Träger 129 verbunden, der an seinem von der Aufnahmeeinrichtung 125 abgewandten Ende mit einem Handgriff 130 oder dergleichen ausgestattet ist.

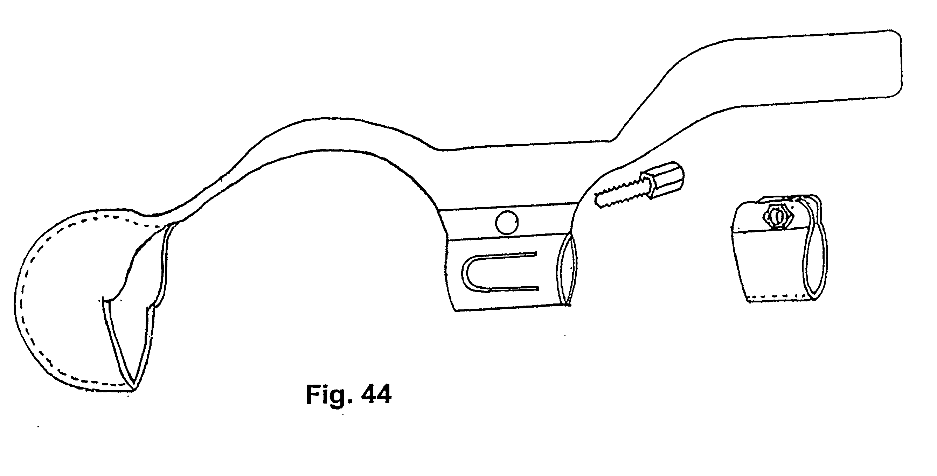

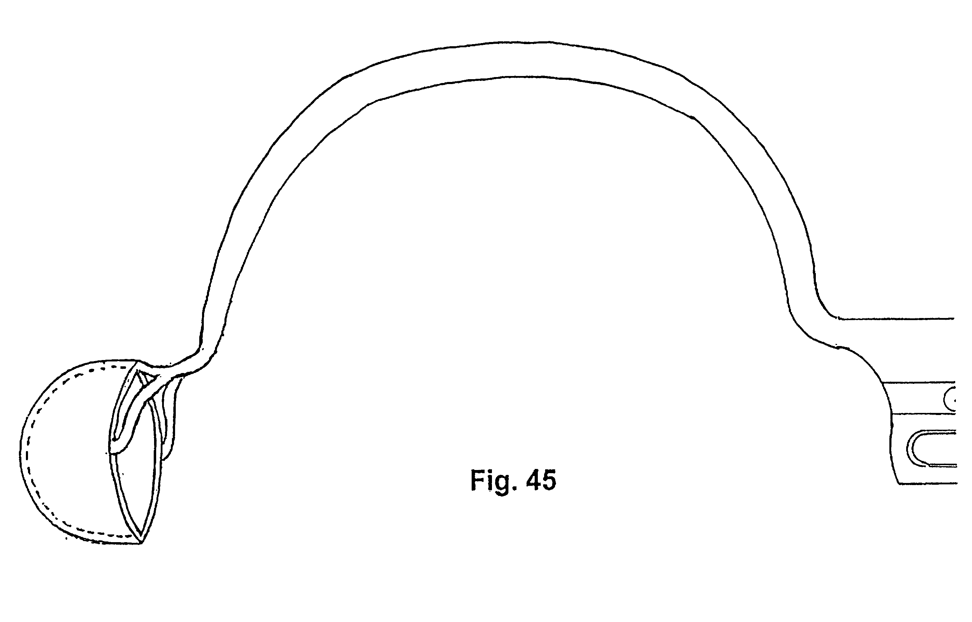

Die Haltestege 128 sind so gebogen, daß nach Überstülpen des Aufnahmeelements 126 über den Femurkopf 121 der zuvor aus der Pfanne des Hüftgelenks dislozierte Femurkopf repositioniert werden kann. Außerdem kann gegebenenfalls der Träger 129 in seinem der Kappe zugewandten Teil bogenförmig ausgebildet sein, um besseren Spielraum zu schaffen, wie dies in Fig. 44 dargestellt ist. Entsprechend Fig. 45 kann auch ein relativ großer Bogen vorgesehen sein, der es erlaubt, eine Bohrung unter Verwendung eines medialen Zugangs und eines Lateralschnittes einzubringen, wobei der Bogen vor dem Oberschenkel liegt.

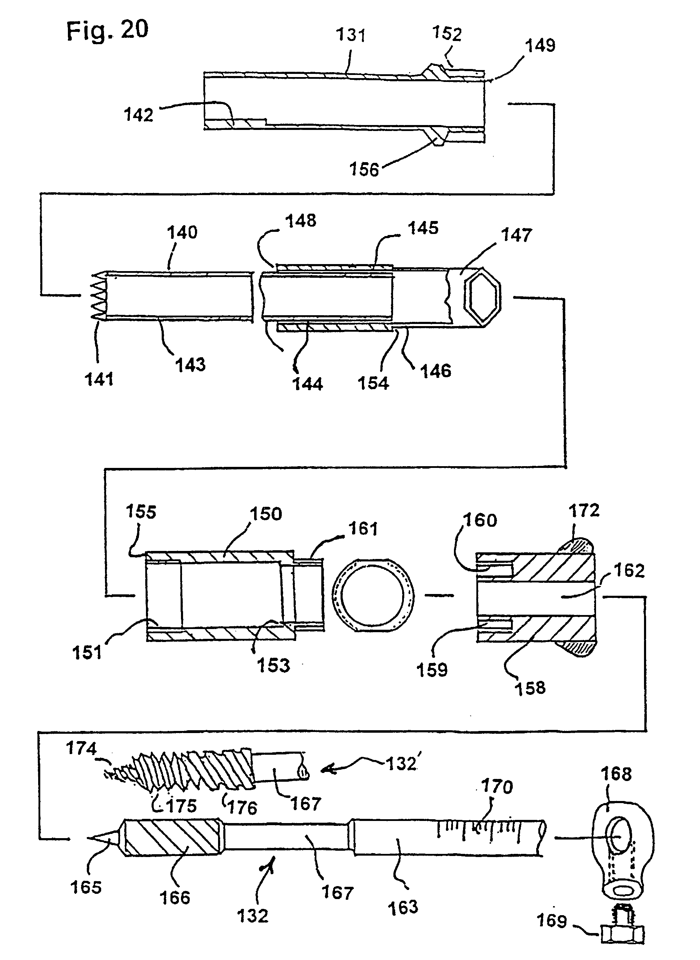

Mit dem seitlichen Träger 129 ist ferner eine Aufnahmebuchse 131 zur Aufnahme eines Bohrers 132 oder eines anderen Instruments verbunden. Bei der veranschaulichten Ausführungsform ist die Aufnahmebuchse 131 durch einen an dem Träger 129 befestigten Rohrstutzen 133 gesteckt. Dem Rohrstutzen 133 ist eine Schelle 134 zugeordnet, die durch Betätigen einer Schraube 135 wahlweise gelöst oder festgezogen werden kann. Das Aufnahmeelement 126 ist der Mittellängsachse der Aufnahmebuchse 131 zentrisch zugeordnet. Nach Lösen der Schraube 135 kann die Aufnahmebuchse 131 gegenüber der Aufnahmeeinrichtung 125 längenverstellt werden. Dabei läßt sich beispielsweise der Axialabstand zwischen dem vorderen Ende der Aufnahmebuchse 131 und dem Lotpunkt 136 der Mittellängsachse des Aufnahmeelements 126 auf dessen Innenfläche 127 an einer Skala 137 ablesen, die auf der Außenfläche der Aufnahmebuchse 131 vorgesehen ist und die zur Abstandsanzeige mit der Hinterkante 138 des Rohrstutzens 133 zusammenwirkt. Durch Anziehen der Schraube 135 und damit der Schelle 134 läßt sich die Aufnahmebuchse 131 mit Bezug auf den Träger 129 und das Aufnahmeelement 126 arretieren. Wie aus den Fign. 19 und 20 hervorgeht, ist in Radialrichtung zwischen der Aufnahmebuchse 131 und dem Bohrer 132 eine Zwischenhülse 140 koaxial zu diesen angeordnet. Die Zwischenhülse 140 steht über die Aufnahmebuchse 131 beidseitig axial über. An dem der Aufnahmeeinrichtung 125 zugewandten Ende trägt die Zwischenhülse 140 einen Domenkranz 141 oder dergleichen. An Stelle des Dornenkranzes 141 können an der Zwischenhülse 140 auch anders geformte spitz zulaufende Vorsprünge oder dergleichen vorgesehen sein.

Um den Femurkopf 121 in der Aufnahmeeinrichtung 125 fest einspannen zu können und somit während des Einbringens der Bohrung 122 die Vorrichtung 120 und den Fernurschaft mit Femurkopf relativ zueinander festzulegen, ist die Zwischenhülse 140 in der Aufnahmebuchse 131 drehgesichert längsverschieblich gelagert. Zur Drehsicherung ist bei der veranschaulichten Ausführungsform ein Nut-Feder-System vorgesehen, zu dem eine von der Innenfläche der Aufnahmebuchse 131 nach innen radial abstehende Feder 142 gehört, die in einem Schlitz oder einer Nut 143 der Zwischenhülse 140 in Längsrichtung verschiebbar geführt ist. Die Zwischenhülse 140 trägt an ihrem von dem Domenkranz 141 abliegenden Ende ein Außengewinde 144, das mit einem Innengewinde 145 einer Verstellbuchse 146 in Gewindeeingriff steht. Die Verstellbuchse 146 weist an dem von der Aufnahmeeinrichtung 125 abgewandten Ende ein Betätigungsteil 147 auf, das im veranschaulichten Ausführungsbeispiel als Hülsenabschnitt mit sechseckigem Querschnitt ausgebildet ist.

Im zusammengebauten Zustand legt sich die Verstellbuchse 146 mit ihrer dem Dornenkranz 141 zugewendeten Stirnfläche 148 gegen die von der Aufnahmeeinrichtung 125 abgewendete Stirnfläche 149 der Aufnahmebuchse 131 an. Die Verstellbuchse 146 wird in dieser Lage von einer Befestigungshülse 150 gehalten, die über ein Innengewinde 151 auf einen Außengewindeabschnitt 152 an dem von der Aufnahmeeinrichtung 125 abgewendeten Ende der Aufnahmebuchse 131 aufgeschraubt ist. Die Befestigungshülse 150 weist einen inneren Anschlag, insbesondere in Form einer Innenschulter 153, auf, der sich gegen einen äußeren Anschlag, im veranschaulichten Beispiel in Gestalt einer Außenschulter 154, der Verstellbuchse 146 anlegt. Auf diese Weise wird die Verstellbuchse 146 drehbar, aber gegen eine axiale Verstellung gegenüber der Aufnahmebuchse 131 und der Befestigungshülse 150 gesichert gehalten. Die Befestigungshülse 150 läßt sich mit Bezug auf die Aufnahmebuchse 131 arretieren, indem ihr der Aufnahmeeinrichtung 125 zugewendetes Ende 155 durch Anziehen der Gewinde 151, 152 gegen einen Steg 156 gepreßt wird, der von der Außenseite der Aufnahmebuchse 131 vorragt und den Außengewindeabschnitt 152 in Richtung auf die Aufnahmeeinrichtung 125 begrenzt. Der Steg 156 kann dabei in der in Fig. 19 angedeuteten Weise am Umfang mit Abflachungen versehen sein, um die Aufnahmebuchse 131 mittels eines Werkzeugs beim Festziehen der Befestigungshülse 150 sicher halten zu können.

Mit der Befestigungshülse 150 ist eine Zentrierbuchse 158 lösbar verbunden. Bei dem Ausführungsbeispiel gemäß den Fign. 19 und 20 ist die Zentrierbuchse 158 für diesen Zweck im Bereich einer Axialnut 159 mit einem Innengewinde 160 versehen, das mit einem Außengewinde 161 der Befestigungshülse 150 zusammenwirkt. Die Zentrierbuchse 158 hat eine Längsbohrung 162 mit einem Innendurchmesser, der dem Außendurchmesser eines Schafts 163 des Bohrers 132 oder eines anderen Instruments derart angepaßt ist, daß Letzteres weitgehend spielfrei aufgenommen wird. Auf diese Weise wird eine exakte Führung des Bohrers 132 beim Eindringen in das Knochensystem erreicht.

Der in Fig. 20 dargestellte Bohrer 132 weist eine Zentrierbohrspitze 165 auf, an die sich eine Schneide 166 anschließt. Ein in Längsrichtung zwischen der Schneide 166 und dem Schaft 163 liegender Abschnitt 167 des Bohrers 132 hat einen kleineren Durchmesser als die Schneide 166. Dadurch wird während des Bohrens die Erzeugung von Reibungswärme klein gehalten. Außerdem nimmt der zwischen der Umfangsfläche des Abschnitts 167 und der Innenwand der Zwischenhülse 140 verbleibende Ringraum Bohrmehl auf, das beim Ausbilden der Bohrung 122 anfällt. Auf den Schaft 163 des Bohrers kann ein Anschlag 168 aufgesetzt werden, der mittels einer Schraube 169 mit Bezug auf den Bohrer 132 fixiert wird. Der Bohrerschaft 163 trägt eine Längenskalierung 170. Durch entsprechendes Einstellen des Anschlags 168 gegenüber der Längenskalierung 170 läßt sich die Bohrtiefe einstellen, z.B. derart, daß dann, wenn der Anschlag 168 an der Zentrierbuchse 158 anstößt, die Zentrierbohrspitze 165 kurz vor dem Lotpunkt 136 steht.

Anhand von vor dem chirurgischen Eingriff hergestellten Röntgenaufnahmen kann die Vorrichtung 120 unter Nutzung der Längenskalierungen 137 und 170 voreingestellt werden. Dabei wird durch Lösen und Wiederanziehen von Schelle 134 und Schraube 135 die Aufnahmebuchse 131 in einen geeigneten Abstand von dem Aufnahmeelement 126 gebracht, während die Bohrtiefe durch Verstellen des Anschlags 168 entlang dem Bohrerschaft 163 eingestellt wird. Die Zwischenhülse 140 ist in der Aufnahmebuchse 131 zurückgeschoben und ragt aus dem der Aufnahmeeinrichtung 125 zugewandten Ende der Aufnahmebuchse 131 nicht oder nur wenig vor. Dann wird das Aufnahmeelement 126 auf den präparierten oder gegebenenfalls unpräparierten Femurkopf 121 in der in Fig. 19 angedeuteten Weise aufgesetzt, wodurch die Mittellängsachse 171 der Aufnahmebuchse 131 und damit auch die Bohrerlängsachse gegenüber dem Femurkopf 121 selbsttätig zentrisch ausgerichtet werden. Dann wird mittels eines zweckentsprechenden Werkzeugs, beispielsweise eines in das Betätigungsteil 147 eingesteckten Imbusschlüssels, die Verstellbuchse 146 gedreht, um durch den Eingriff zwischen den Gewinden 144 und 145 die Zwischenhülse 140 in Richtung auf das Aufnahmeelement 126 zu verschieben, bis der Domenkranz 141 in festem Eingriff mit dem Femurschaft 123 steht. Die Gewinde 144, 145 sind zweckmäßig als linksgängige Gewinde ausgebildet. Die Vorrichtung 120 wird auf diese Weise am Knochensystem sicher befestigt. Jetzt wird die Zentrierbuchse 158 an die Befestigungshülse 150 angeschraubt, und der Bohrer wird mit eingestellter Bohrtiefe so weit vorangeschoben, bis seine Spitze Knochenkontakt hat. Dann wird der Bohrer 132 mittels einer zweckentsprechenden Antriebsquelle, beispielsweise eines pneumatischen Antriebes, in Drehung versetzt und in Richtung auf das Aufnahmeelement 126 vorgetrieben, bis der Anschlag 168 an der Zentrierbuchse 158 anschlägt. Dabei wird der Bohrer durch das Zusammenwirken zwischen dem Bohrerschaft 163 und der Zentrierbuchse 158 genau geführt, während aufgrund des Abschnitts 167 mit reduziertem Durchmesser die Entwicklung von Reibungswärme im Bohrkanal kleingehalten wird. Die Zentrierbuchse 158 läßt sich bei Verwendung eines Werkzeuges mit anderem Außendurchmesser gegen eine Zentrierbuchse mit entsprechend angepaßtem Bohrungsdurchmesser auswechseln. Ein solcher Wechsel wird durch Griffe oder Ansätze 172 an der Zentrierbuchse erleichtert (Fig. 20).

In Fig. 20 ist bei 132' ein abgewandelter Bohrer veranschaulicht, der an seiner Spitze ein konisch zulaufendes Schneideteil 174 aufweist, an das ein kurzes Schraubengewindeteil anschließt, auf das ein Hauptschneideteil 176 folgt. An das Hauptschneideteil 176 schließt wiederum ein Abschnitt 167 von verringertem Durchmesser an. Das Schraubengewindeteil 175 sorgt für einen Vortrieb des Bohrers 132' entlang dem Bohrkanal, so daß der Antrieb des Bohrers im wesentlichen nur für eine Drehantriebskraft zu sorgen braucht.

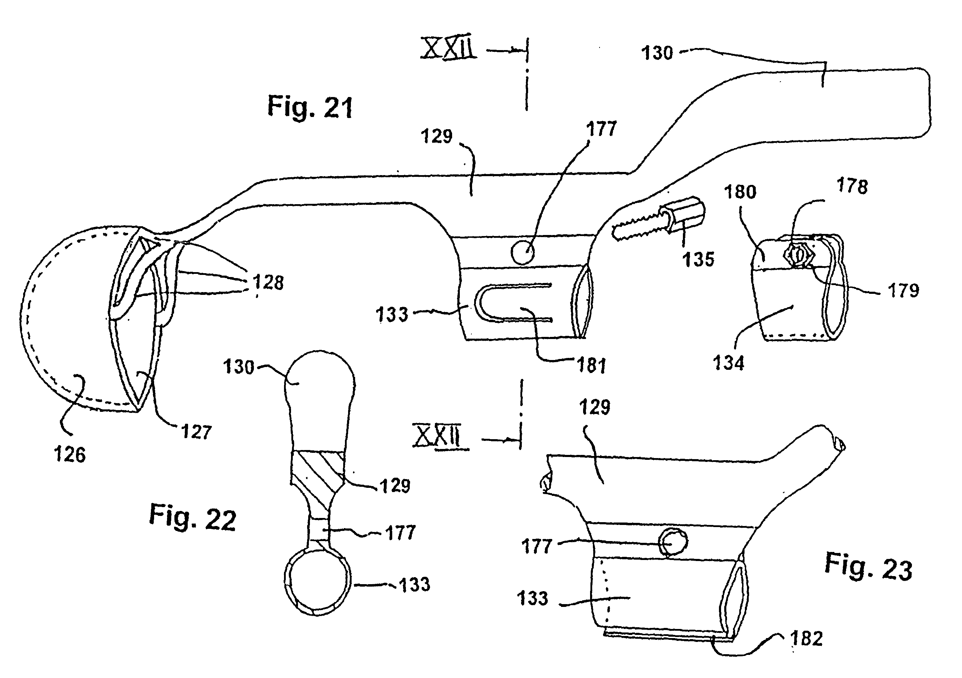

In Fig. 21 sind der Rohrstutzen 133, die Schelle 134 und die Schraube 135 auseinandergezogen dargestellt. Im zusammenmontierten Zustand durchgreift die Schraube 135 miteinanderausgerichtete Bohrungen 177 und 178 des Trägers 129 bzw. der Schelle 134, und sie ist in eine Mutter 179 eingeschraubt, die an einem Schenkel 180 der Schelle 134 befestigt ist. Um die auf die Aufnahmebuchse 131 wirkende Klemmkraft zu übertragen, ist in dem Rohrstutzen 133 auf einander diametral gegenüberliegenden Seiten jeweils eine Federzunge 181 ausgebildet. Stattdessen oder zusätzlich kann der Rohrstutzen 133 beispielsweise auch mit einem durchgehenden Längsschlitz 182 versehen sein, wie dies in Fig. 23 gezeigt ist.

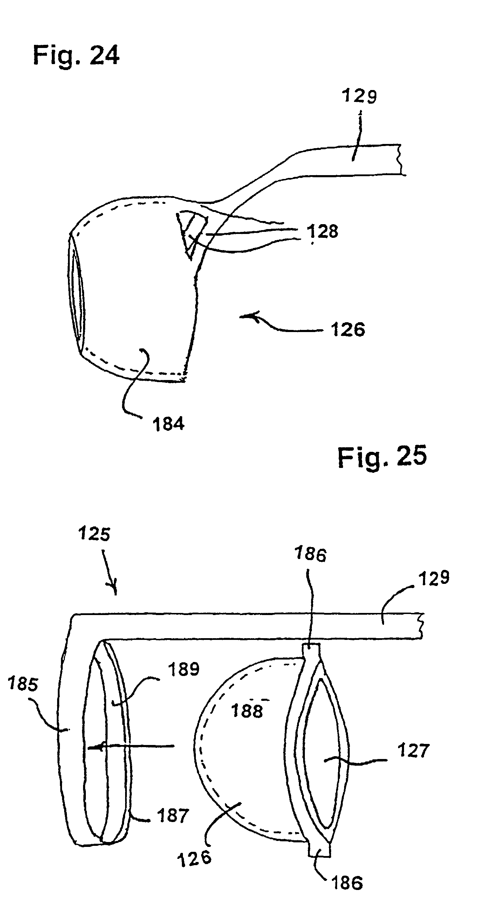

Bei der Ausführungsform gemäß Fig. 24 wird das Aufnahmeelement 126 von einem Flächenband 184 gebildet, dessen Innenform gleichfalls an die Außenform des Femurkopfes 121 so angepaßt ist, daß seine Innenfläche an wenigstens einem Teil der Außenfläche des präparierten oder unpräparierten Femurkopfes zur Anlage gebracht werden kann. Die Breite des Flächenbandes 184 kann in weiten Grenzen variiert werden. Es muß lediglich gewährleistet sein, daß durch das Zusammenwirken zwischen der Innenfläche des Flächenbandes 184 und der Außenfläche des Femurkopfes eine exakte Ausrichtung der Mittellängsachse der Aufnahmebuchse 131 in Relation zu der Peripherie des Gelenkkopfes und eine entsprechende Positionierung der Vorrichtung 120 gegenüber dem Knochensystem erfolgen.

Bei der weiter abgewandelten Ausführungsform der Aufnahmeeinrichtung 125 gemäß Fig. 25 ist an dem Träger 129 eine beispielsweise ringförmige Halteeinrichtung 185 angebracht, die mit dem Aufnahmeelement 126 lösbar verbunden werden kann. Das Aufnahmeelement 126 ist in Fig. 25 als Kappe dargestellt. Stattdessen kann beispielsweise auch ein Flächenband entsprechend dem Flächenband 184 nach Fig. 24 vorgesehen sein. Das Aufnahmeelement 126 kann in verschiedenen Größen entsprechend verschieden großen Femurköpfen vorgesehen sein und mit radial nach außen stehenden Vorsprüngen 186 oder beispielsweise einem Ringbund an einer Haltefläche 187 der Halteeinrichtung 185 zur Anlage gebracht werden. Das Aufnahmeelement 126 kann über nicht näher dargestellte Befestigungseinrichtungen in der Halteeinrichtung 185 lösbar fixiert werden. Als Befestigungseinrichtung eignet sich beispielsweise eine Rast-, Schnapp- oder dergleichen -einrichtung oder eine Schraubverbindung zwischen der Außenfläche 188 des Aufnahmeelements 126 und der Innenumfangsfläche und/oder der Haltefläche 187 der Halteeinrichtung 185.

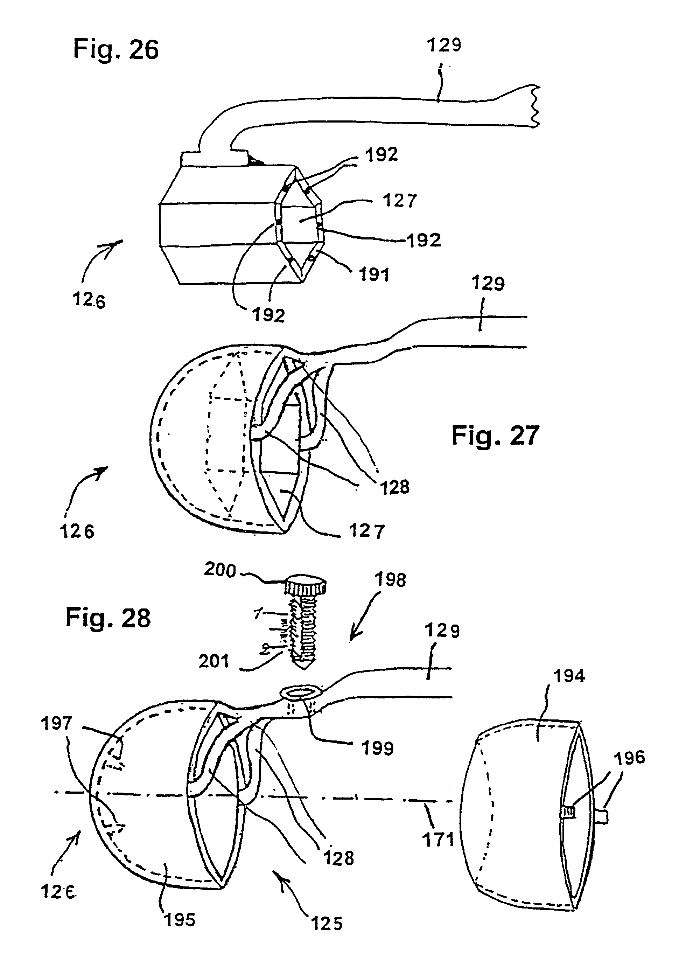

Die in den Fign. 26 und 27 dargestellten Ausführungsformen des Aufnahmeelements 126 weisen einen Innenraum mit sechseckigem Querschnitt auf, wobei das Aufnahmeelement 126 in Fig. 26 als Hülse und in Fig. 27 als Kappe ausgebildet ist. Ein solches Aufnahmeelement wird sich an drei oder mehr Stellen seiner Innenfläche 127 und/oder Stirnfläche 191 gegen den betreffenden Gelenkkopf anlegen, beispielsweise an drei oder mehr Stellen der Stirnfläche 191, die entsprechend Fig. 26 Auflager 192 darstellen, welche in diesem Fall in einer Ebene angeordnet sind. Es versteht sich, daß das Aufnahmeelement 126 auch von einem anders gestalteten, z.B. drei-, vier- oder sonstigen vieleckigen Körper gebildet sein kann. Eine genaue Positionierung und Justierung des Gelenkkopfes gegenüber der Mittellängsachse der Aufnahmebuchse 131 ist durch wenigstens drei Auflager 192 des Aufnahmeelements 126 gewährleistet, die mit der Peripherie des Gelenkkopfes zusammenwirken. Auch Hohlzylinder, Hohlkegel und anders geformte Körper sind als Aufnahmeelement grundsätzlich geeignet, sofern sie den Gelenkkopf mindestens teilweise aufnehmen und sich gegen dessen Außenfläche bzw. Peripherie an mindestens drei trigonal angeordneten Stellen anlegen können.

Fig. 28 zeigt eine weitere Ausgestaltung der Aufnahmeeinrichtung 125, bei welcher als Teil des Aufnahmeelements 126 und/oder der Aufnahmeeinrichtung 125 ein Einsatz 194 vorgesehen ist, der aus einer Gruppe von Einsätzen unterschiedlicher Wandstärke und/oder Innenform zur Anpassung des Innendurchmessers und/oder der Innenform des Aufnahmeelements an den jeweiligen Durchmesser und/oder die Außenform des Gelenkkopfes ausgewählt und in einen trägerfesten Teil 195 des Aufnahmeelements lösbar und austauschbar eingesetzt wird. Um den Austausch zu erleichtern, kann der Einsatz 194 mit abstehenden Nasen 196 versehen sein. Ein solcher Einsatz kann mit besonderem Vorteil auch bei allen anderen Ausführungsformen der erläuterten Vorrichtung verwendet werden.

Gegebenenfalls kann das Aufnahmeelement 126, wie ebenfalls in Fig. 28 angedeutet ist, an seiner Innenseite ein oder mehrere Drehsicherungselemente 197, beispielsweise in Form von Dornen oder dergleichen, tragen. Auch kann eine Justiervorrichtung 198 zum Einstellen des Abstandes des Trägers 129 von der Mittellängsachse 171 der Aufnahmebuchse 131 vorgesehen sein. Bei der in Fig. 28 dargestellten Ausführungsform weist eine solche Justiervorrichtung eine in den Träger 129 integrierte Innengewindebuchse 199 auf, deren Achse im wesentlichen senkrecht zu der Längsmittelachse des Aufnahmeelements 126 steht. In die Innengewindebuchse 199 ist eine Justierschraube 200 einschraubbar, die mit einer Skalierung 201 versehen sein kann.

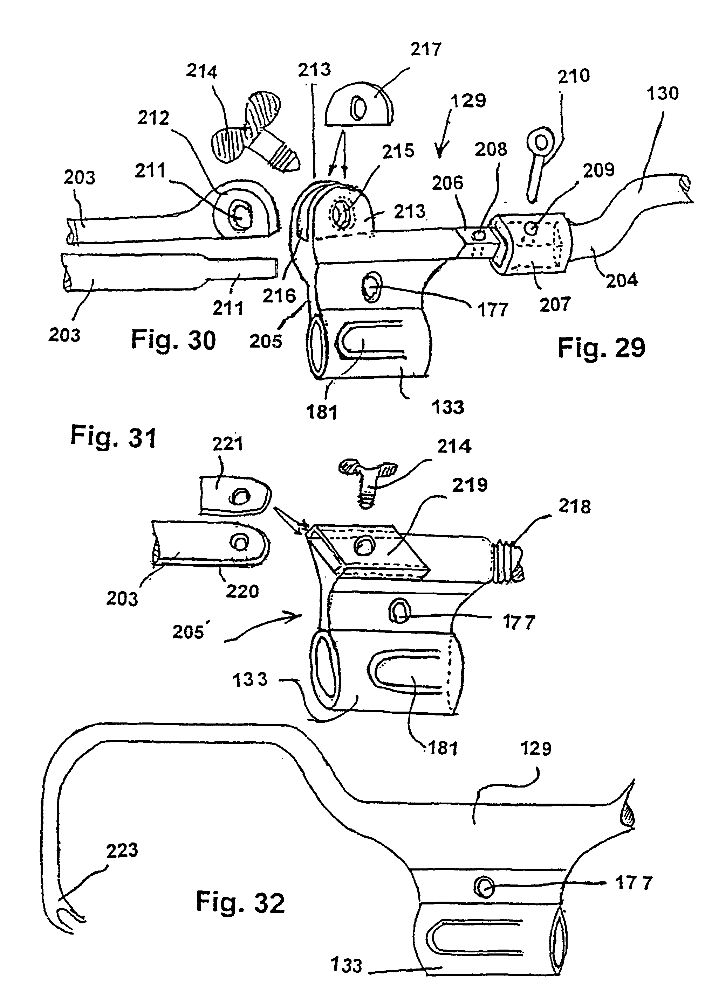

Entsprechend den Fign. 29 bis 31 können ein die Aufnahmeeinrichtung 125 tragender Teil 203 und/oder ein den Handgriff 130 aufweisender Teil 204 des Trägers 129 mit dem restlichen Teil der Vorrichtung lösbar verbunden sein. Für diesen Zweck weist der Träger 129 beispielsweise einen den Rohrstutzen 133 tragenden Abschnitt 205 auf, der mit einem Steckansatz 206 in eine Aufnahme 207 des Trägerteils 204 einsteckbar ist, wobei der Steckansatz 206 und die Aufnahme 207 eine komplementäre polygonale, z.B. quadratische, Außenform bzw. Innenform haben. Der Steckansatz 206 und die Aufnahme 207 sind mit Bohrungen 208 bzw. 209 versehen, die nach dem Zusammenstecken miteinander fluchten und in die ein Splint 210, eine Halteschraube oder dergleichen einsteckbar bzw. einschraubbar ist. Das Trägerteil 203 ist bei dem Ausführungsbeispiel gemäß den Fign. 29 und 30 mit einem eine durchgehende Bohrung 211 aufweisenden Kopf 212 versehen, der zwischen zwei parallel in Abstand voneinander angeordnete Haltenasen 213 des Abschnittes 205 eingelegt und dort mittels einer Flügelschraube 214 fixiert werden kann. Die Flügelschraube 214 erstreckt sich im zusammengebauten Zustand durch eine der Bohrung 211 entsprechende Bohrung in der einen der Haltenasen 213, und sie ist in eine entsprechende Gewindebohrung 215 der anderen Haltenase 213 eingeschraubt. Die Breite des Kopfes 212 kann im wesentlichen gleich der Breite des von den Haltenasen 213 begrenzten, der Aufnahme des Kopfes 212 dienenden Spalts 216 gewählt sein. Der Kopf 212 kann aber auch schmaler als der Spalt 216 gehalten sein, und in den Spalt 216 kann auf der einen oder der anderen Seite des Kopfes 212 zusätzlich eine Distanzscheibe 217 eingesteckt und gleichfalls von der Schraube 214 gehalten sein. Im letztgenannten Fall wird je nach dem, auf welcher Seite des Kopfs 212 die Distanzscheibe 217 eingelegt wird, die Zentralachse der Aufnahmeeinrichtung 125 in einem vorbestimmten Abstand parallel zu der Mittellängsachse der Aufnahmebuchse 131 auf der einen oder der anderen Seite dieser Mittellängsachse angeordnet. Es kann auf diese Weise einfach für eine genau vordefinierte Exzentrizität zwischen der Zentralachse der Aufnahmeeinrichtung und der Mittellängsachse der Aufnahmebuchse 131 gesorgt werden.

Fig. 31 zeigt eine abgewandelte Ausführungsform des Trägerabschnittes, die mit 205' bezeichnet ist und an Stelle des Steckansatzes 206 einen Außengewinde tragenden Ansatz 218 trägt, mit welchem der den Handgriff 130 aufweisende Teil 204 des Trägers 129 verschraubt werden kann. Der Abschnitt 205' trägt an seiner in Fig. 31 oben liegenden Seite eine hülsenförmige Aufnahme 219, in welche das von der Aufnahmeeinrichtung 125 abliegende Ende 220 des Trägerteils 203 eingesteckt werden kann. Zur gegenseitigen Fixierung von Trägerteil 203 und Abschnitt 205' kann wiederum eine Flügelschraube 214 vorgesehen sein, die durch entsprechende Öffnungen der Aufnahme 219 und des Endes 220 gesteckt und in eine in Fig. 31 nicht veranschaulichte Gewindebohrung des Abschnitts 205' eingeschraubt wird. Hat das Ende 220 des Trägerteils 203 eine Stärke entsprechend der Höhenabmessung der Aufnahme 219, liegt die Zentralachse der Aufnahmeeinrichtung 125 gleichachsig zu der Mittellängsachse 171 der Aufnahmebuchse 131. Wird die Stärke des Endes 220 jedoch kleiner als die Höhenabmessung der Aufnahme 219 gehalten, kann ähnlich wie bei dem Ausführungsbeispiel gemäß Fig. 29 eine Distanzscheibe 221 zusammen mit dem Ende 220 auf dessen einer oder anderer Seite in die Aufnahme 219 eingesteckt werden, um für eine genau vordefinierte gegenseitige Versetzung zwischen der Zentralachse der Aufnahmeeinrichtung und der Längsmittelachse der Aufnahmebuchse zu sorgen.

Der Träger 129 kann gemäß der abgewandelten Ausführungsform der Fig. 32 auch mit einem Fühler 223 lösbar oder, wie dargestellt, einstückig verbunden sein. Der Fühler 223 kann als Kondylenfühler oder dergleichen ausgebildet sein.

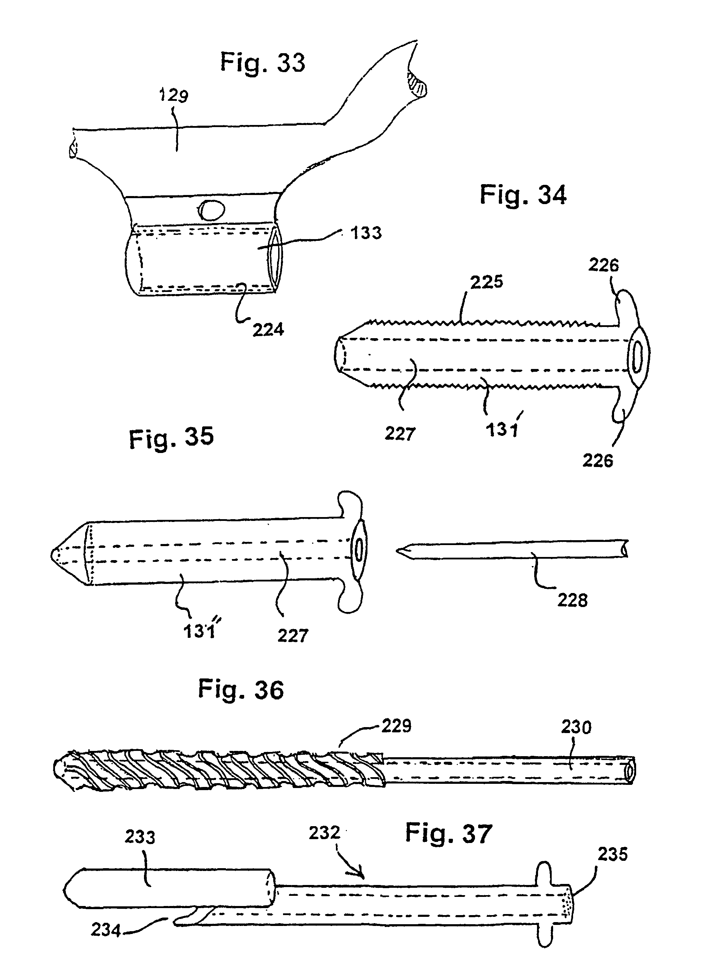

Bei der abgewandelten Ausführungsform gemäß Fig. 33 ist der mit dem Träger 129 verbundene Rohrstutzen 133 mit einem Innengewinde 224 versehen. In die Gewindebohrung des Rohrstutzens 133 kann eine in Fig. 34 dargestellte Aufnahmebuchse 131' mit einem Außengewinde 225 eingeschraubt werden, wobei die Aufnahmebuchse 131' zur bequemen Betätigung an dem von der Aufnahmeeinrichtung 125 abliegenden Ende flügelschraubenförmige Ansätze 226 trägt. Bei dieser Ausführungsform kann die Zwischenhülse 140 weggelassen werden. Durch entsprechend weites Einschrauben kann die Aufnahmebuchse 131' selbst zur Anlage an dem Knochensystem gebracht werden, um die Vorrichtung 120 mit Bezug auf das Knochensystem festzulegen. Wegen des Wegfalls der Zwischenhülse 140 können auch die Verstellbuchse 146 und die Befestigungshülse 150 weggelassen werden. Wird der Durchmesser der Längsbohrung 227 dem Werkzeugdurchmesser angepaßt, kann des weiteren auch die Zentrierbuchse 158 entfallen.

Fig. 35 zeigt eine Aufnahmebuchse 131" der vorstehend anhand der Fig. 34 erläuterten Art mit einer Längsbohrung 227, deren Durchmesser einem Kirschnerdraht 228 angepaßt ist. Unter Verwendung der Aufnahmebuchse 131" der Fig. 35 kann dabei mittels der Vorrichtung 120 zunächst der Kirschnerdraht 228 achsengerecht in das Knochensystem eingeschossen werden. Die Vorrichtung 120 kann dann von dem in dem Knochensystem verbleibenden Kirschnerdraht 228 abgezogen werden, und ein in Fig. 36 dargestellter Hohlbohrer 229 mit einer dem Außendurchmesser des Drahtes 228 angepaßten Innenlängsbohrung 230 kann geführt durch den Kirschnerdraht 228 in das Knochensystem eingetrieben werden.

Fig. 37 zeigt ein meißel- oder hobelförmiges Instrument 232 mit einem Führungsbolzen 233, der nach Einbringen der Bohrung 122 in das von dem Gelenkkopf abgewendete Ende dieser Bohrung eingesteckt werden kann. Das Instrument 232 ist ferner mit einer Schneide 234 versehen. Mittels der Schneide 234 kann das von dem Gelenkkopf abliegende Ende der Bohrung 122 bearbeitet werden, indem auf das Ende 235 des Instruments 232 geschlagen wird. Insbesondere kann so ein Bett zum Einbringen der Blattfeder 22 oder einer an Stelle einer solchen Feder verwendeten Winkelplatte genau und einfach ausgebildet werden.

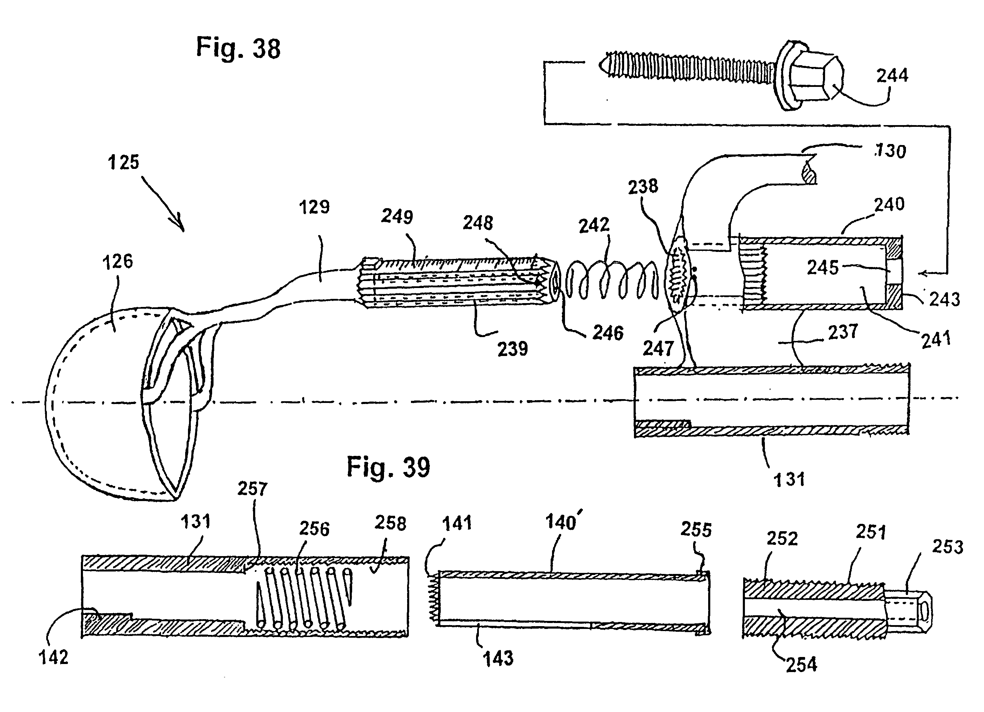

Fig. 38 zeigt eine weiter abgewandelte Ausführungsform der chirurgischen Vorrichtung, bei welcher der die Aufnahmeeinrichtung 125 und die Aufnahmebuchse 131 untereinander verbindende seitliche Träger 129 mit Bezug auf die Aufnahmebuchse 131 dreh- und längenverstellbar, jedoch in der eingestellten Drehlage drehgesichert ist. Für diesen Zweck weist ein mit der Aufnahmebuchse 131 fest verbundener Trägerabschnitt 237 eine Kerbzahnnabe 238 auf, die mit einer Kerbzahnwelle 239 an dem von dem Aufnahmeelement 126 abgewandten Ende des Trägers 129 zusammenwirkt. Die Kerbzahnnabe 238 ist Teil einer Buchse 240, die eine Kammer 241 zur Aufnahme einer Druckfeder 242 bildet. Die Druckfeder 242 stützt sich mit ihrem einen Ende an der Stirnfläche der Kerbzahnwelle 239 und mit ihrem anderen Ende an einem Boden 243 der Buchse 240 ab. Eine Stellschraube 244 erstreckt sich im zusammenmontierten Zustand durch eine Mittelöffnung 245 des Bodens 243, die Druckfeder 242 und die Kerbzahnnabe 238 hindurch in eine Gewindebohrung 246, die koaxial zu der Längsachse der Kerbzahnwelle 239 verläuft. Bei Anziehen der Stellschraube 244 wird die Aufnahmeeinrichtung 125 entgegen der Kraft der Feder 242 in Richtung auf die Aufnahmebuchse 131 gezogen. Wird die Stellschraube 244 in der entgegengesetzten Richtung gedreht, erfolgt unter dem Einfluß der Feder 242 ein Verstellen der Aufnahmeeinrichtung 125 weg von der Aufnahmebuchse 131. Nach Lösen der Stellschraube 244 kann die Kerbzahnwelle 239 auch ganz aus der Kerbzahnnabe 238 herausgezogen und in einer anderen Drehstellung mit Bezug auf die Kerbzahnnabe wieder in diese eingesetzt werden, um für eine gegenseitige Versetzung der Zentralachse des Aufnahmeelements 126 und der Längsmittelachse der Aufnahmebuchse 131 zu sorgen. Diejenige Winkelausrichtung der Kerbzahnwelle 239 mit Bezug auf die Kerbzahnnabe 238, in welcher die Zentralachse des Aufnahmeelements 126 mit der Längsmittelachse der Aufnahmebuchse 131 ausgerichtet ist, kann zweckmäßig durch Markierungen 247 und 248 an der Außenseite der Kerbzahnnabe 238 bzw. der Kerbzahnwelle 239 kenntlich gemacht sein. Im veranschaulichten Ausführungsbeispiel trägt die Kerbzahnwelle 239 ferner eine Längenskalierung 249.

Fig. 39 zeigt eine Ausführungsform der chirurgischen Vorrichtung, bei welcher eine Buchse 251 die Funktion sowohl der Verstellbuchse 146 als auch der Zentrierbuchse 158 der Ausführungsform gemäß den Fign. 19 und 20 übernimmt. Die Buchse 251 weist einen Außengewindeabschnitt 252 und einen Betätigungsteil 253 auf. Der Durchmesser einer Längsmittelbohrung der Buchse 251 ist dem Außendurchmesser des zu verwendenden Werkzeugs (insbesondere Bohrer oder Draht) angepaßt, um für eine weitgehend spielfreie Führung zu sorgen. Die Zwischenhülse 140' weist in diesem Fall nicht das Außengewinde 144 auf, sondern sie trägt an ihrem von dem Dornenkranz 141 abgewandten Ende am Außenumfang einen Ringanschlag 255. Im zusammengebauten Zustand legt sich der Ringanschlag 255 gegen das eine Ende einer Druckfeder 256 an, die in die Aufnahmebuchse 131 eingesetzt ist und den an den Ringanschlag 255 angrenzenden Teil der Zwischenhülse 140 umgreift. Dabei legt sich das andere Ende der Druckfeder 256 gegen einen Ringanschlag 257 am Innenumfang der Aufnahmebuchse 131 an. Der Außengewindeabschnitt 252 der Buchse 251 wirkt mit einem Innengewindeabschnitt 258 der Aufnahmebuchse 131 zusammen. Durch entsprechend weites Einschrauben der Buchse 251 in die Aufnahmebuchse 131 läßt sich der Dornenkranz 141 der Zwischenhülse 140 in Eingriff mit demKnochensystem bringen, und die Vorrichtung in der oben erläuterten Weise zu fixieren.

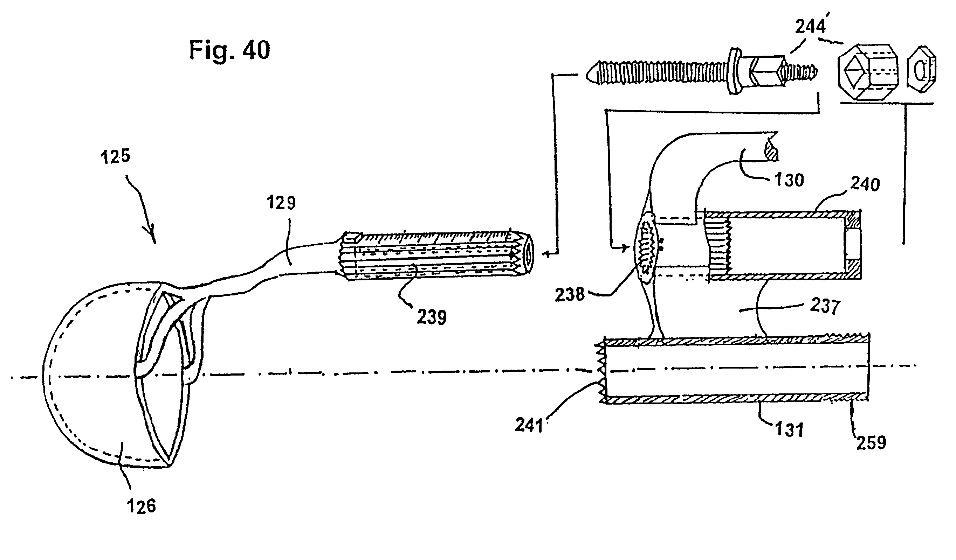

Die in Fig. 40 veranschaulichte Vorrichtung entspricht hinsichtlich der einstellbaren Verbindung von Aufnahmeeinrichtung 125 und Aufnahmebuchse 131 der Ausführungsform gemäß Fig. 38. Die Aufnahmebuchse 131 ist in diesem Fall jedoch selbst mit dem Dornenkranz 141 versehen, und die Fixierung der Vorrichtung am Knochensystem erfolgt durch Betätigen der Stellschraube 244', wobei die Feder 242 entfällt, aber die Stellschraube 244' zur Längenverstellung zwischen Aufnahmebuchse und Aufnahmeelement dient. Die Zentrierbuchse 158 kann auf einen Gewindeabschnitt 259 der Aufnahmebuchse 131 aufgeschraubt werden. Der Innendurchmesser der Aufnahmebuchse 131 kann aber auch selbst dem Außendurchmesser des zu verwendenden Werkzeugs angepaßt sein. Bei einer solchen Ausgestaltung kann auch die Zentrierbuchse entfallen.

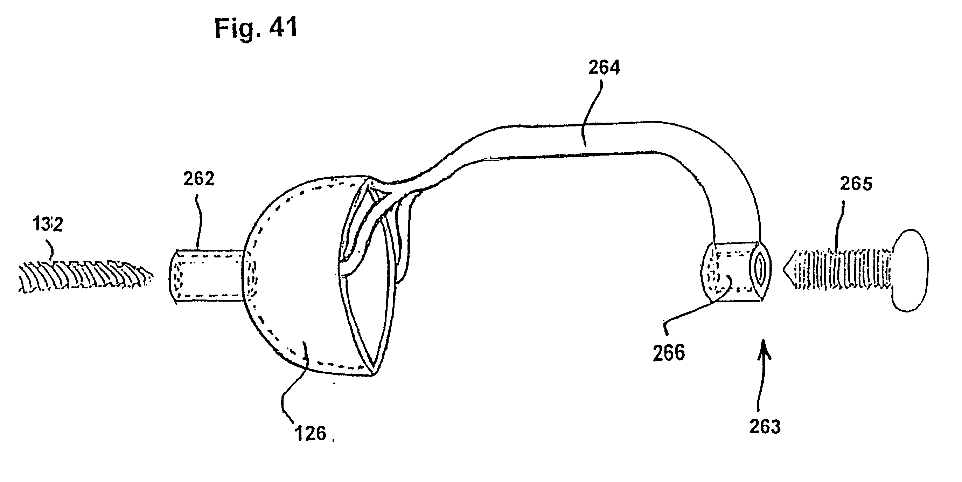

In Fig. 41 ist eine weiter abgewandelte Ausführungsform der chirurgischen Vorrichtung veranschaulicht, bei welcher das Aufnahmeelement 126 eine Aufnahmebuchse 262 aufweist. Die Aufnahmebuchse 262 steht in Richtung der Längsmittelachse des Aufnahmeelements 126 von der Seite des Aufnahmeelements ab, die von einem Halteelement 263 abgewendet ist. Das Aufnahmeelement 126 und das Halteelement 263 sind über einen seitlichen Träger 264 miteinander verbunden, dessen mittlerer Teil zugleich als Handgriff benutzt werden kann. Das Halteelement 263 weist bei der dargestellten Ausführungsform eine Fixierschraube 265 auf, die in eine Gewindebuchse 266 des Trägers 264 eingeschraubt ist. Die Aufnahmebuchse 262 führt ein Werkzeug, beispielsweise in Form des Bohrers 132, wobei in diesem Fall die Bohrung von der Gelenkkopfseite aus eingebracht wird.

Es versteht sich, daß zahlreiche weitere Abwandlungen möglich sind. Beispielsweise können auch bei den Ausführungsbeispielen der Fign. 38, 40 und 41 Drehsicherungselemente 197 an der Innenseite des Aufnahmeelements 126 vorgesehen sein. Der Schaft des Bohrers und die Innenfläche des Führungsteils der Zentrierbuchse bzw. der Aufnahmebuchse können jeweils mit einem Schraubengewinde versehen sein, wobei durch den gegenseitigen Eingriff dieser Gewinde dafür gesorgt wird, daß eine Drehung des Bohrers selbsttätig zu einer Längsverstellung desselben führt. Des weiteren kann die Aufnahmebuchse an dem Träger über eine einstellbare Führungsbolzen- und Hülseneinrichtung angebracht sein.

Was die Prothese anbelangt, kann das Biegeelement 40 dem kappenseitigen oder dem halteteilseitigen Teleskopelement zugeordnet sein. Es können auch mehrere Biegeelemente vorhanden sein. Auf die Gelenkverbindung zwischen der Teleskopanordnung und der Befestigungsplatte kann gegebenenfalls verzichtet werden, oder es können Mittel vorgesehen sein, um eine vorhandene Gelenkverbindung temporär zu versteifen, beispielsweise während der Heilungsperiode nach Oberschenkelhalsfrakturen oder bei Osteoporöse. Für eine solche Versteifung kann beispielsweise dadurch gesorgt werden, daß an die Befestigungsplatte 16 eine Sperrplatte angesetzt wird, die ein Steckelement trägt, das in das halteteilseitige Ende der Teleskopanordnung einsteckbar ist.

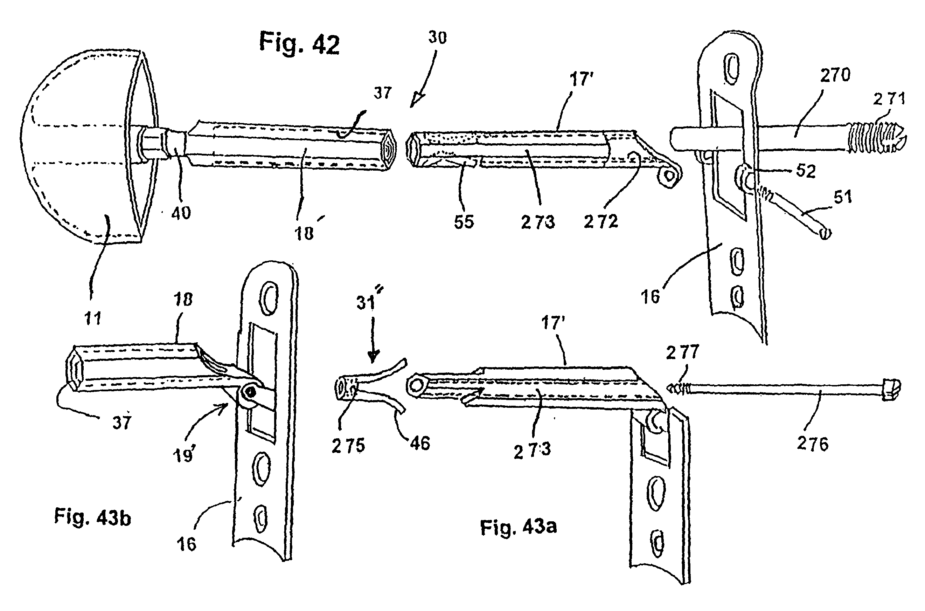

Bei der in Fig. 42 dargestellten Ausführungsform der Gelenkprothese ist entsprechend Fig. 4 das Aufnahmeelement 18' kappenseitig angeordnet. Jedoch fehlt der Kanal 49. Um die als Rasteinrichtung ausgebildete Kupplungseinrichtung 30 von der Halteteilseite her bedarfsweise in und außer Eingriff bringen zu können, sind an dem Steckelement 17' Federarme 55 gemäß Fig. 5 angebracht, die sich mittels eines von der Halteseite her zu betätigenden Schraubstifts 270 ausstellen lassen. Der Schraubstift 270 trägt für diesen Zweck an seinem in Fig. 42 rechten Ende ein Außengewinde 271, das mit einem Innengewindeteil 272 eines durchgehenden Axialkanals 273 des Steckelements 17' in Eingriff kommt. Durch Eindrehen des Schraubstifts 270 werden die Federarme 55 ausgespreitzt; sie kommen dadurch mit Rastausnehmungen der beispielsweise sägezahnartig oder anders profilierten beziehungsweise unprofilierten Anlagefläche 37 an der Innenseite des Aufnahmeelements 18' in Eingriff. Wird dagegen der Schraubstift 270 herausgedreht, verstellen sich die Federarme 55 unter dem Einfluß von Federvorspannkraft selbsttätig nach innen und damit außer Eingriff mit den Rastausnehmungen der Anlagefläche 37.

Bei der Ausführungsform nach Fig. 43a ist eine Feder 31" ähnlich der Feder 31' der Fig.2, jedoch mit einem zusätzlichen Innengewindeabschnitt 275, vorgesehen. Die Federarme 46 der Feder 31 " lassen sich aufspreizen, indem von der Halteteilseite her ein Schraubstift 276 in den Axialkanal 273 des Steckelements 17' eingebracht und mit einem Außengewindeabschnitt 277 in den Innengewindeabschnitt 275 eingeschraubt wird.

Die Fig. 43b zeigt ein Ausführungsbeispiel, bei dem zur Bildung des Drehgelenks 19' Gelenkarme von der Befestigungsplatte 16 in Richtung auf das Aufnahmeelement 18 vorstehen. Mit diesen Gelenkarmen steht das Aufnahmeelement 18 in Scharnierverbindung. Diese Ausführungsform erlaubt es, das Drehgelenk in der äußeren Mündung der Bohrung versenkt anzuordnen.

Zur Herstellung des Aufnahmeelements 18 bzw. 18' kann beispielsweise von einer zylindrischen Hülse mit kreisrundem Querschnitt ausgegangen werden, in die ein Innengewinde eingeschnitten wird. Die Hülse kann dann zu einem Mehrkant, beispielsweise Sechskant, geformt werden.

## Patentansprüche

1. Gelenkprothese für den mit einer Pfanne zusammenwirkenden Gelenkkopf eines gelenks, insbesondere eines Hüftgelenks,
- mit einer Kappe (11,11',11",111), die eine Anlagefläche (13) zur Anlage der Kappe an dem Gelenkkopf und eine mindestens näherungsweise kugelkappenförmige Außenfläche (12) aufweist,
- mit einer Teleskopanordnung (15), die ein mit der Kappe (11,11',11",111), verbundenes und von der Kappe vorragendes kappenseitiges Teleskopelement 17, 17'', 17''', 18', 117, 117', 117'' und ein damit in Teleskopeingriff stehendes halteteilseitiges Teleskopelement 17', 18, 18'', 118 aufweist und die so bemessen und angeordnet ist, daß sie sich im implantierten Zustand durch eine Bohrung in dem Gelenkkopf und daran anschließende Knochenbereiche hindurcherstreckt, sowie
- mit einem Halteteil (16), das mit dem halteteilseitigen Teleskopelement 17', 18, 18'', 118 in Verbindung steht und das zur Befestigung an einer dem Gelenkkopf gegenüberliegenden Knochenfläche ausgebildet ist,
wobei das kappenseitige Teleskopelement 17, 17'', 17''', 18', 117, 117', 117'' gegenüber dem halteteilseitigen Teleskopelement 17', 18, 18'', 118 in der von der Pfanne wegweisenden Richtung verschiebbar geführt ist,
**dadurch gekennzeichnet, daß**
- die Anlagefläche von einer der Form des präparierten oder unpräparierten Gelenkkopfes mindesten näherungsweise angepaßten Innenfläche (13) der Kappe (11, 11', 11 ", 111) gebildet ist, und
- eine richtungsabhängig wirkende Hemmvorrichtung (30, 42, 67) vorgesehen ist, die eine Verstellung der Kappe gegenüber dem Halteteil (16) in der von der Pfanne wegweisenden Richtung erlaubt und in der auf die Pfanne zuweisenden Richtung verhindert oder mindestens im Vergleich zu einer Verstellung in der von der Pfanne wegweisenden Richtung erschwert.

2. Gelenkprothese nach Anspruch 1, **dadurch gekennzeichnet, daß** die Teleskopanordnung (15) flexibel und/oder gelenkig ausgebildet und/oder die Teleskopanordnung mit der Kappe (11, 11', 11", 111) und/oder dem Halteteil (16) flexibel und/oder gelenkig verbunden ist, wodurch die Gelenkprothese im implantierten Zustand die biomechanischen Bewegungen des Gelenkkopfes und Knochenhalses gegenüber dem Schaft des betreffenden Knochens mindestens teilweise zuläßt.

3. Gelenkprothese nach Anspruch 1 oder 2, **dadurch gekennzeichnet, daß** das kappenseitige Teleskopelement ein Steckelement (17, 17", 17''', 117, 117") ist und das halteteilseitige Teleskopelement als Aufnahmeelement (18, 18", 118) ausgebildet ist.

4. Gelenkprothese nach Anspruch 1 oder 2, **dadurch gekennzeichnet, daß** das halteteilseitige Teleskopelement ein Steckelement (17') ist und das kappenseitige Teleskopelement als Aufnahmeelement (18') ausgebildet ist.

5. Gelenkprothese nach einem der vorhergehenden Ansprüche, **dadurch gekennzeichnet, daß** die Innenfläche (13) der Kappe (11, 11', 11", 111) mindestens näherungsweise kugelkappenförmig ausgebildet ist.

6. Gelenkprothese nach einem der vorhergehenden Ansprüche, **dadurch gekennzeichnet, daß** als Halteteil eine Befestigungsplatte (16) vorgesehen ist, die mit dem von der Kappe (11, 11', 11", 111) abgewandten Ende des halteteilseitigen Teleskopelements (17', 18, 18", 118) verbunden ist.

7. Gelenkprothese nach Anspruch 6, **dadurch gekennzeichnet, daß** das halteteilseitige Teleskopelement (17', 18, 18", 118) mit der Befestigungsplatte (16) beweglich verbunden ist.

8. Gelenkprothese nach Anspruch 7, **dadurch gekennzeichnet, daß** das halteteilseitige Teleskopelement (17', 18, 18", 118) an der Befestigungsplatte (16) angelenkt ist.

9. Gelenkprothese nach Anspruch 8, **dadurch gekennzeichnet, daß** das halteteilseitige Teleskopelement (17', 18, 18", 118) mit der Befestigungsplatte (16) über ein Drehgelenk (19) verbunden ist, dessen Drehachse im wesentlichen senkrecht zu der Längserstrekkung der Teleskopanordnung (15) und im wesentlichen senkrecht zu der Längserstrekkung der Befestigungsplatte angeordnet ist.

10. Gelenkprothese nach Anspruch 8 oder 9, **dadurch gekennzeichnet, daß** das halteteilseitige Teleskopelement (118) mit der Befestigungsplatte (16) über ein Drehgelenk (77) verbunden ist, dessen Drehachse im wesentlichen senkrecht zu der Längserstreckung der Teleskopanordnung (15) und im wesentlichen parallel zu der Längserstreckung der Befestigungsplatte angeordnet ist.

11. Gelenkprothese nach einem der vorhergehenden Ansprüche, **dadurch gekennzeichnet, daß** das halteteilseitige Teleskopelement (17', 18, 18", 118) an dem Halteteil (16) federelastisch abgestützt ist.

12. Gelenkprothese nach einem der Ansprüche 7 bis 11, **gekennzeichnet durch** ein Sperrelement zum wahlweisen temporären Versteifen der beweglichen Verbindung zwischen dem halteteilseitigen Teleskopelement (17', 18, 18", 118) und der Befestigungsplatte (16).

13. Gelenkprothese nach Ansprüchen 11 und 12, **dadurch gekennzeichnet, daß** zum federelastischen Abstützen des halteteilseitigen Teleskopelements (17', 18, 18", 118) eine sich an das halteteilseitige Teleskopelement und an die Befestigungsplatte (16) anlegende Blattfeder (22) vorgesehen ist, und daß das Sperrelement als Winkelplatte ausgebildet ist, die bedarfsweise gegen die Blattfeder austauschbar ist.

14. Gelenkprothese nach einem der vorhergehenden Ansprüche, **dadurch gekennzeichnet, daß** die Teleskopanordnung (15) mindestens ein die biomechanischen Bewegungen des Gelenkkopfes mindestens teilweise zulassendes Biegeelement (40) aufweist.

15. Gelenkprothese nach Anspruch 14, **dadurch gekennzeichnet, daß** das Biegeelement (40) in einem Abstand von der Innenfläche (13) der Kappe (11, 11', 11", 111 ) angeordnet ist, der dem etwa ein- bis dreifachen, insbesondere dem ungefähr eineinhalbfachen, des Innenradius der Kappe entspricht.

16. Gelenkprothese nach Anspruch 14 oder 15, **dadurch gekennzeichnet, daß** das Biegeelement (40) verdrehsicher ausgebildet ist.

17. Gelenkprothese nach einem der Ansprüche 14 bis 16, **dadurch gekennzeichnet, daß** das Biegeelement (40) als torsionsfeste Feder, biegsame Welle, Geflechtwelle, Federgelenk, Einsatz oder dergleichen ausgebildet ist.

18. Gelenkprothese nach einem der Ansprüche 14 bis 16, **dadurch gekennzeichnet, daß** das Biegeelement (40) mit dem kappenseitigen Teleskopelement (17, 17", 17"',18', 117, 117') einstückig verbunden ist.

19. Gelenkprothese nach einem der vorhergehenden Ansprüche, **dadurch gekennzeichnet, daß** mindestens eines der beiden Teleskopelemente (17"') aus zwei oder mehr in Längsrichtung der Teleskopanordnung im wesentlichen hintereinander liegenden Teilen (60, 61) besteht, die ineinander drehfest einsteckbar oder einschraubbar und gegen selbsttätiges Lösen sicherbar sind.

20. Gelenkprothese nach einem der vorhergehenden Ansprüche, **dadurch gekennzeichnet, daß** die Hemmvorrichtung als selbsttätig richtungsabhängig wirksam gemachte Kupplungseinrichtung (30), insbesondere Rast- oder Schnappeinrichtung, ausgebildet ist.

21. Gelenkprothese nach Anspruch 20, **dadurch gekennzeichnet, daß** die Kupplungseinrichtung (30) mindestens einen vorstehenden Rastvorsprung (32, 63) an einem der Teleskopelemente (17, 17', 18", 117) oder einem deren Teile (61) aufweist, der derart geformt und/oder mit Federkraft beaufschlagt ist, daß er bei einer versuchten Verstellung des kappenseitigen Teleskopelements bzw. -teils gegenüber dem halteteilseitigen Teleskopelement bzw. -teil in der auf die Pfanne zuweisenden Richtung mit einer Anlagefläche (37, 58) des anderen Teleskopelements bzw. -teils (17" 18, 18', 60) in Sperreingriff kommt, eine Verstellung des kappenseitigen Teleskopelements bzw. -teils gegenüber dem halteteilseitigen Teleskopelement bzw. -teil in von der Pfanne wegweisender Richtung jedoch zuläßt.

22. Gelenkprothese nach Anspruch 21, **dadurch gekennzeichnet, daß** der Rastvorsprung (32, 63) von mindestens einer radial nach außen oder innen wirkenden Feder (31, 31', 55, 59) gebildet ist, die mit Bezug auf das eine Teleskopelement oder -teil (17, 17', 17''' 18, 18", 117) festgelegt ist.

23. Gelenkprothese nach Anspruch 21, **dadurch gekennzeichnet, daß** der Rastvorsprung (32) von mindestens einer radial nach außen oder innen wirkenden Feder (55, 59) gebildet ist, die mit dem einen Teleskopelement oder -teil (117, 18") einstückig verbunden ist.

24. Gelenkprothese nach einem der Ansprüche 21 bis 23, **gekennzeichnet durch** einen über den Rastvorsprung (32) schiebbaren Ring (47), der den Rastvorsprung zunächst zurückhält und erst dann selbsttätig freigibt, wenn die beiden Teleskopelemente bzw. -teile ineinandergeschoben werden.

25. Gelenkprothese nach einem der Ansprüche 21 bis 24, **dadurch gekennzeichnet, daß** die Anlagefläche (37, 58) des anderen Teleskopelements bzw. -teils (17" 18, 18', 60) mindestens eine Rastausnehmung (64), insbesondere eine Mehrzahl von Rastausnehmungen in Form eines sägezahnartigen Profils oder dergleichen, aufweist.

26. Gelenkprothese nach einem derAnsprüche 21 bis 24, **dadurch gekennzeichnet, daß** die Anlagefläche des anderen Teleskopelements bzw. -teils unprofiliert ausgebildet und/oder von einem Überzug gebildet ist, in den sich der Rastvorsprung einkrallen kann.

27. Gelenkprothese nach einem der vorhergehenden Ansprüche, **gekennzeichnet durch** eine Federvorspanneinrichtung (42, 67), welche das kappenseitige Teleskopelement (17, 117') in der von der Pfanne wegweisenden Richtung vorspannt.

28. Gelenkprothese nach Anspruch 27, **dadurch gekennzeichnet, daß** die Federvorspanneinrichtung mindestens eine Zugfeder (42) aufweist, die einerseits mit dem kappenseitigen Teleskopelement (17) und andererseits mit dem halteteilseitigen Teleskopelement (18) und/oder dem Halteteil (16) verbunden ist.

29. Gelenkprothese nach Anspruch 27 oder 28, **dadurch gekennzeichnet, daß** die Federvorspanneinrichtung (67) zugleich die richtungsabhängig wirkende Hemmvorrichtung bildet.

30. Gelenkprothese nach Anspruch 29, **dadurch gekennzeichnet, daß** das als Steckelement ausgebildete Teleskopelement (117') eine Außenschulter (71) und das als Aufnahmeelement ausgebildete Teleskopelement (118) eine Innenschulter (72) aufweist und eine Vorspannfeder (67) zwischen den beiden Schultern sitzt.

31. Gelenkprothese nach Anspruch 30, **dadurch gekennzeichnet, daß** das als Steckelement ausgebildete Teleskopelement (117') von zwei im wesentlichen gleichachsig angeordneten und miteinander lösbar verbundenen, insbesondere miteinander verschraubten, Teilen (68, 69) gebildet ist, von denen das eine (68) mit der Kappe (11) verbunden ist und das andere (69) die Außenschulter (71 ) trägt.

32. Gelenkprothese nach einem der vorhergehenden Ansprüche, **gekennzeichnet durch** eine Anordnung (35, 35', 69, 270, 276), die es erlaubt, die Hemmvorrichtung zur Demontage der Prothese unwirksam zu machen.

33. Gelenkprothese nach einem der vorhergehenden Ansprüche, **dadurch gekennzeichnet, daß** sich das kappenseitige Teleskopelement (17, 17''' 18', 117, 117') im wesentlichen zentrisch von der Innenfläche (13) der Kappe (11, 11', 11", 111) wegerstreckt.

34. Gelenkprothese nach einem der vorhergehenden Ansprüche, **gekennzeichnet durch** mindestens eine Drehsicherung zum Verhindern einer Drehung der Kappe (11, 11', 11", 111) gegenüber dem Gelenkkopf.

35. Gelenkprothese nach Anspruch 34, **dadurch gekennzeichnet, daß** die beiden Teleskopelemente (17, 18; 17', 18'; 17", 18"; 18, 117; 117', 118) in drehfester gegenseitiger Verbindung stehen.

36. Gelenkprothese nach Anspruch 35, **dadurch gekennzeichnet, daß** der Querschnitt der beiden Teleskopelemente (17, 18; 17', 18'; 17", 18"; 18, 117; 117', 118) so profiliert ist, daß für die drehfeste gegenseitige Verbindung gesorgt ist.

37. Gelenkprothese nach einem der Ansprüche 34 bis 36, **dadurch gekennzeichnet, daß** das kappenseitige Teleskopelement (17) in einem vorbestimmten Abstand von der Mittelachse bzw. der Peripherie der Kappe (11) exzentrisch angeordnet ist.

38. Gelenkprothese für den mit einer Pfanne zusammenwirkenden Femurkopf eines Hüftgelenks,
- mit einer Kappe (11), die eine Anlagefläche (13) zur Anlage der Kappe an dem Gelenkkopf und mindestens eine näherungsweise kugelkappenförmige Außenfläche aufweist, und
- mit mindestens einem zur Befestigung an einer dem Gelenkkopf gegenüberliegenden Knochenfläche ausgebildeten Halteteil (16),
**dadurch gekennzeichnet, daß**
- die Anlagefläche von einer der Form des präparierten oder unpräparierten Gelenkkopfes mindestens näherungsweise angepaßten Innenfläche (13) der Kappe (11) gebildet ist; und
- mindestens eine Hemmvorrichtung (93) vorgesehen ist, die eine gegenseitige Verstellung zwischen der Kappe und dem Halteteil (16) in der auf die Pfanne zuweisenden Richtung im Vergleich zu einer Verstellung in der von der Pfanne wegweisenden Richtung erschwert, wobei die Hemmvorrichtung mindestens ein, insbesondere federelastisches, Zugelement (94) aufweist, das sich im implantierten Zustand durch eine Bohrung in dem Gelenkkopf und daran anschließenden Knochenteilen hindurcherstreckt., und wobei das Zugelement einerseits mit der Kappe und andererseits mit dem Halteteil verbunden ist und die Kappe in der von der Pfanne wegweisenden Richtung vorspannt.

39. Gelenkprothese nach Anspruch 38, **dadurch gekennzeichnet, daß** sich von der Innenfläche der Kappe (11) mindestens ein Stiel (96) wegerstreckt und das Zugelement (94) an dem von der Kappeninnenfläche (13) abliegenden Ende des Stieles angreift.

40. Gelenkprothese nach einem der vorhergehenden Ansprüche, **gekennzeichnet durch** eine Hülse, die in die Bohrung einsetzbar ist und die im implantierten Zustand die Teleskopanordnung bzw. das Zugelement und/oder den Stiel über mindestens einen Teil ihrer Längsabmessung umschließt.

41. Gelenkprothese nach einem der Ansprüche 38 bis 40, **gekennzeichnet durch** mindestens eine Drehsicherung (50; 100, 101) zum Verhindern einer Drehung der Kappe (11) gegenüber dem Gelenkkopf.

42. Gelenkprothese nach einem der Ansprüche 34 bis 41, **dadurch gekennzeichnet, daß** die Kappe (11) an ihrer Innenfläche mindestens ein Drehsicherungselement (50; 100, 101) zum Verhindern einer Drehung der Kappe gegenüber dem Gelenkkopf trägt.

43. Gelenkprothese nach einem der Ansprüche 34 bis 42, **dadurch gekennzeichnet, daß** die Kappe mit mindestens einer exzentrisch angeordneten Bohrung (100) versehen ist, in die ein zum Eindringen in den Gelenkkopf bestimmtes Drehsicherungselement (101) von außen einbringbar und versenkbar ist.

44. Gelenkprothese nach einem der Ansprüche 39 bis 43, **dadurch gekennzeichnet, daß** der Stiel (96, 102) zentrisch oder in einem vorbestimmten Abstand von der Mittelachse bzw. der Peripherie der Kappe (11) exzentrisch angeordnet ist.

45. Gelenkprothese nach einem der vorhergehenden Ansprüche, **dadurch gekennzeichnet, daß** die Teleskopanordnung oder der Stiel wenigstens im Bereich der Kappe außenseitig einen kreisrunden Querschnitt aufweist.

46. Gelenkprothese nach einem der vorhergehenden Ansprüche, **dadurch gekennzeichnet, daß** der Rand (81 ) der Kappe (11") mindestens zu einem größeren Teil in einer Ebene liegt, die derart schiefwinklig zu der Längsachse der Prothese steht, daß der Gelenkkopf von der Kappe im implantierten Zustand mindestens näherungsweise gleichmäßig überdeckt wird.

47. Gelenkprothese nach einem der vorhergehenden Ansprüche, **dadurch gekennzeichnet, daß** die Kappe (111) mit Ausnehmungen (84) versehen ist, die vorzugsweise an der Außenfläche der Kappe entgratet oder trichterförmig ausgebildet sind.

48. Gelenkprothese nach einem der vorhergehenden Ansprüche, **gekennzeichnet durch** mindestens einen als Innenteil der Kappe in die Kappe einsetzbaren Einsatz (86), dessen Innenfläche als Anlagefläche der Form des präparierten oder unpräparierten Gelenkkopfes mindestens näherungsweise angepaßt ist und der aus einer Gruppe von Einsätzen unterschiedlicher Wandstärke und/oder Form zur Anpassung des Innendurchmessers und/oder der Innenform der Kappe an den Durchmesser und/oder die Außenform des jeweiligen Gelenkkopfes ausgewählt und eingesetzt wird.

49. Gelenkprothese nach Anspruch 48, **dadurch gekennzeichnet, daß** der Einsatz (86) mit einer zentrischen und/oder einer exzentrischen Aussparung (88) zum Durchstecken des kappenseitigen Teleskopelements bzw. des Stiels und eventuell eines Drehsicherungselements versehen ist.

50. Gelenkprothese nach Ansprüchen 40 und 48 oder 49, **dadurch gekennzeichnet, daß** der Einsatz und die sich über mindestens einen Teil der Längsabmessung der Teleskopanordnung bzw. des Stiels erstreckende Hülse miteinander fest verbunden sind.

51. Gelenkprothese nach einem der vorhergehenden Ansprüche, **dadurch gekennzeichnet, daß** Teil der Kappe ein Kappenoberteil (89) ist, das auf einem Kappenunterteil (90) drehbar gelagert ist.

52. Gelenkprothese nach einem der vorhergehenden Ansprüche, **dadurch gekennzeichnet, daß** der Pol und/oder Unterpol (80) der Kappe (11') abgeflacht ist.

53. Gelenkprothese nach einem der vorhergehenden Ansprüche, **gekennzeichnet durch** eine Abdeckung und/oder Polsterung des Halteteils an der von der Knochenanlagefläche abgewendeten Seite.

54. Gelenkprothese nach Anspruch 8 oder 9, **dadurch gekennzeichnet, daß** das Gelenk (19') zur versenkten Anordnung in der Bohrung ausgebildet ist.

## Claims

1. Joint prosthesis for a joint head of a joint, particularly a hip joint, which head cooperates with a joint socket, the prosthesis comprising:
- a cap (11, 11', 11",111) having a contact surface (13) with which the cap abuts the joint head, and having an outer surface (12) which is at least approximately shaped as a spherical cap,
- a telescopic arrangement (15) having a cap-side telescopic member (17, 17", 17''', 18', 117, 117', 117") linked with the cap and projecting from the cap, and a supporting member-side telescopic member (17', 18, 18", 118) which is in telescopic engagement with the cap-side telescopic member, the telescopic arrangement being sized and arranged so as to pass through a drilling in the joint head and bone regions adjacent thereto when in an implanted state, and
- a supporting member (16) attached to the supporting member-side telescopic member (17',18, 18", 118) and designed for fixation to a bone surface located opposite to the joint head,
wherein the cap-side telescopic member (17, 17", 17''', 18', 117, 117', 117") is guided for sliding movement in relation to the supporting member-side telescopic member (17', 18, 18", 118) in a direction away from the joint socket,
**characterized in that**
- the contact surface is defined by an internal surface (13) of the cap (11, 11', 11",111), which internal surface is at least approximately adapted to the shape of the prepared or unprepared joint head, and
- directionally operative restraining means (30, 42, 67) is provided which permits changes in position of the cap with respect to the supporting member (16) in the direction away from the joint socket, and which at least impedes changes in position of the cap with respect to the supporting member in the direction towards the joint socket in comparison with changes of position in the direction away from the joint socket.

2. Joint prosthesis according to claim 1 **characterized in that** the telescopic arrangement (15) is flexible and/or articulated and/or the telescopic arrangement is flexibly or articulatedly connected to the cap (11, 11', 11", 111) and/or to the supporting member (16), whereby the joint prosthesis, in its implanted state, at least partly permits the biomechanical movements of the joint head and joint neck relative to the shaft of the respective bone.

3. Joint prosthesis according to claim 1 or 2 **characterized in that** the cap-side telescopic member is a male member (17, 17", 17''' 117, 117") and the supporting member-side telescopic member is designed as a female member (18, 18", 118).

4. Joint prosthesis according to claim 1 or 2 **characterized in that** the supporting member-side telescopic member is a male member (17') and the cap-side telescopic member is designed as a female member (18').

5. Joint prosthesis according to any one of the preceding claims **characterized in that** the internal surface (13) of the cap (11, 11', 11" 111) is at least approximately shaped like a spherical cap.

6. Joint prosthesis according to any one of the preceding claims **characterized in that** the supporting member is comprised of a supporting plate (16) which is attached to the end of the supporting member-side telescopic member (17', 18, 18'', 118) remote from the cap (11, 11',.11" 111).

7. Joint prosthesis according to claim 6 **characterized in that** the supporting member-side telescopic member (17', 18, 18", 118) is pivotally connected to the supporting plate (16).

8. Joint prosthesis according to claim 7 **characterized in that** the supporting member-side telescopic member (17', 18, 18", 118) is connected to the supporting plate (16) by a joint.

9. Joint prosthesis according to claim 8 **characterized in that** the supporting member-side telescopic member (17', 18, 18", 118) is connected to the supporting plate (16) by means of a hinge (19) the hinge axis of which is substantially perpendicular to the longitudinal extension of the telescopic arrangement (15)and substantially perpendicular to the longitudinal extension of the supporting plate.

10. Joint prosthesis according to claim 8 or 9 **characterized in that** the supporting member-side telescopic member (118) is connected to the supporting plate (16) by means of a hinge (77) the hinge axis of which is substantially perpendicular to the longitudinal extension of the telescopic arrangement (15) and substantially parallel to the longitudinal extension of the supporting plate.

11. Joint prosthesis according to any one of the preceding claims **characterized in that** the supporting member-side telescopic member (17', 18, 18", 118) is elastically spring-supported on the supporting member (16).

12. Joint prosthesis according to any one of claims 7 to 11 **characterized by** a locking member for selectively temporarily stiffening of the movable coupling between the supporting member-side telescopic member (17', 18, 18", 118) and the supporting plate (16).

13. Joint prosthesis according to claims 11 and 12 **characterized in that** a leaf spring (22) leaning against the supporting member-side telescopic member (17', 18, 18", 118) and the supporting plate (16) is provided for elastically spring-supporting the supporting member-side telescopic member (17', 18, 18", 118), and that the locking member is comprised of an angled plate adapted to be exchanged, if required, against the leaf spring.

14. Joint prosthesis according to any one of the preceding claims **characterized in that** the telescopic arrangement (15) is equipped with at least one flexible member (40) that at least partially permits biomechanical movements of the joint head.

15. Joint prosthesis according to claim 14 **characterized in that** the flexible member (40) is placed at a distance from the inner surface (13) of the cap (11, 11', 11", 111) which distance corresponds to about one to three times, preferably to about one and a half times, the inner radius of the cap.

16. Joint prosthesis according to claim 14 or 15 **characterized in that** the flexible member (40) is designed to prevent twisting motion.

17. Joint prosthesis according to any one of claims 14 to 16 **characterized in that** the flexible member (40) is designed as a spring adapted to prevent torsion, a flexible shaft, a wire mesh shaft, a spring joint, a flexible insert or the like.

18. Joint prosthesis according to any one of claims 14 to 16 **characterized in that** the flexible member (40) is integrally connected to the cap-side telescopic member (17, 17", 17"', 18', 117, 117').

19. Joint prosthesis according to any one of the preceding claims **characterized in that** at least one of the telescopic members (17''') is made of at least two parts (60, 61) disposed substantially one behind the other in the longitudinal direction of the telescopic arrangement, the parts being fitted into each other or screwed together in a manner preventing twisting motion, and being adapted to be secured against spontaneous releasing.

20. Joint prosthesis according to any one of the preceding claims **characterized in that** the restraining means is comprised of automatic, direction-dependent coupling means (30), particularly lock-in or snap-in means.

21. Joint prosthesis according to claim 20 **characterized in that** the coupling means (30) has at least one projecting locking protrusion (32, 63) disposed on one of the telescopic members (17, 17", 18", 117) or on one of the parts (61) thereof, the protrusion being formed and/or spring-biased so as to lockingly engage a contacting surface (37, 58) on the other one of the telescopic members or parts, respectively (17'', 18, 60), when the cap-side telescopic member or part, respectively, is attempted to be displaced relative to the supporting member-side telescopic member or part, respectively, in the direction towards the socket, but to allow displacement of the cap-side telescopic member or part, respectively, relative to the supporting member-side telescopic member or part, respectively, in the direction away from the socket.

22. Joint prosthesis according to claim 21 **characterized in that** the locking protrusion (32, 63) consists of at least one radially outwardly or inwardly acting spring (31, 31', 55, 59) which is in fixed connection with the one telescopic member or part (17, 17', 17''', 18, 18'', 117).

23. Joint prosthesis according to claim 21 **characterized in that** the locking protrusion (32) consists of at least one radially outwardly or inwardly acting spring (55, 59) which is integrally connected with the one telescopic member or part (117, 18").

24. Joint prosthesis according to any one of claims 21 to 23 **characterized by** a ring (47) adapted to be pulled over the locking protrusion (32) for at first holding back the protrusion and for automatically releasing the protrusion upon the telescopic members or parts, respectively, being inserted into each other.

25. Joint prosthesis according to any one of claims 21 to 24 **characterized in that** the contacting surface (37, 58) of the other telescopic member or part (17", 18, 18', 60) comprises at least one snap-in recess (64), preferably a plurality of snap-in recesses in the form of a saw-teeth shaped profile or the like.

26. Joint prosthesis according to any one of claims 21 to 24 **characterized in that** the contacting surface of the other telescopic member or part is without any profile and/or is defined by a coating into which the protrusion is adapted to dig itself

27. Joint prosthesis according to any one of the preceding claims **characterized by** spring biasing means (42, 67) for biasing the cap-side telescopic member (17, 117') in the direction away from the socket.

28. Joint prosthesis according to claim 27 **characterized in that** the spring biasing means comprises at least one tension spring (42) which on the one hand is connected with the cap-side telescopic member (17) and which on the other hand is connected with the supporting member-side telescopic member (18) and/or to the supporting member (16).

29. Joint prosthesis according to claim 27 or 28 **characterized in that** the spring biasing means (67) also defines the restraining means the operation of which is direction-dependent.

30. Joint prosthesis according to claim 29 **characterized in that** the male telescopic member (117') has an external shoulder (71), and the female telescopic member (118) has an internal shoulder (72), and that a biasing spring (67) is disposed between the shoulders.

31. Joint prosthesis according to claim 30 **characterized in that** the male telescopic member (117') is defined by a pair of substantially uniaxially disposed and detachably interconnected, preferably screw-connected, parts (68, 69) one (68) of which is attached to the cap (11) and the other (69) of which is provided with the external shoulder (71 ).

32. Joint prosthesis according to any one of the preceding claims **characterized by** means (35, 35', 69, 270, 276) for disabling the restraining means when disassembling the prosthesis.

33. Joint prosthesis according to any one of the preceding claims **characterized in that** the cap-side telescopic member (17, 17''', 18', 117, 117') substantially centrically projects form the internal surface (13) of the cap (11, 11', 11'', 111).

34. Joint prosthesis according to any one of the preceding claims **characterized by** at least one anti-twist means for preventing rotary motion of the cap (11, 11', 11", 111) relative to the joint head.

35. Joint prosthesis according to claim 34 **characterized in that** the two telescopic members (17, 18; 17', 18'; 17", 18''; 18, 117; 117', 118) are interconnected in a manner preventing relative twisting of the telescopic members.

36. Joint prosthesis according to claim 35 **characterized in that** the cross-sections of the telescopic members (17, 18; 17', 18'; 17", 18"; 18, 117; 117', 118) are profiled to provide for twist-proof mutual interconnection of the telescopic members.

37. Joint prosthesis according to one of claims 34 to 36 **characterized in that** the cap-side telescopic member (17) is disposed eccentrically at a predetermined distance from the central axis or periphery, respectively, of the cap (11).

38. Joint prosthesis for the femoral head of a hip joint, which head cooperates with a socket, the prosthesis comprising:
- a cap (11) having a contact surface (13) with which the cap abuts the joint head, and having an outer surface which is at least approximately shaped as a spherical cap, and
- at least one supporting member (16) designed for fixation to a bone surface located opposite to the joint head,
**characterized in that**
- the contact surface is defined by an internal surface (13) of the cap (11), which internal surface is at least approximately adapted to the shape of the prepared or unprepared joint head, and
- at least one restraining means (93) impeding relative movement between the cap and the supporting member (16) in the direction towards the socket in comparison with relative movement in the direction away of the socket is provided, the restraining means having at least one, preferably spring-elastic, traction member (94) arranged so as to pass through a drilling in the joint head and bone regions adjacent thereto when in an implanted state, the traction member being connected on the one hand to the cap and on the other hand to the supporting member, and the traction member biasing the cap in the direction away from the socket.

39. Joint prosthesis according to claim 38 **characterized in that** at least one stem (96) projects from the inner surface of the cap (11), and **in that** the traction member (94) engages the end of the stem averted from the inner surface (13) of the cap.

40. Joint prosthesis according to any one of the preceding claims **characterized by** a sleeve adapted to be inserted into the drilling, the sleeve embracing the telescopic arrangement along at least a portion of its longitudinal dimension, when the prosthesis is in an implanted state

41. Joint prosthesis according to any one of claims 38 to 40 **characterized by** at least one anti-twist means (50; 100, 101) for preventing rotary motion of the cap (11) relative to the joint head.

42. Joint prosthesis according to any one of claims 34 to 41 **characterized in that** the cap (11) is equipped at its internal surface with at least one anti-twist member (50; 100, 101) for preventing rotary motion of the cap relative to the joint head.

43. Joint prosthesis according to any one of claims 34 to 42 **characterized in that** the cap is equipped with at least one eccentric drilling (100) adapted to receive and imbed from the outside of the cap an anti-rotation member (101) adapted to penetrate into the joint head.

44. Joint prosthesis according to any one of claims 39 to 43 **characterized in that** the stem (96, 102) is disposed centrically or eccentrically at a predefined distance from the central axis or from the periphery, respectively, of the cap (11).

45. Joint prosthesis according to any one of the preceding claims **characterized in that** the telescopic arrangement or the stem has a circular outer perimeter at least for the length thereof extending through the cap.

46. Joint prosthesis according to any one of the preceding claims **characterized in that** at least a major portion of the rim (81) of the cap (11'') lies in a plane that is inclined with respect to the longitudinal axis of the prosthesis at such an angle, that the joint head is at least approximately uniformly covered by the cap, when implanted.

47. Joint prosthesis according to any one of the preceding claims **characterized in that** the cap (111) is provided with recesses (84) which preferably are deburred at the outer surface of the cap or which are funnel-shaped.

48. Joint prosthesis according to any one of the preceding claims **characterized by** at least one insert (86) adapted to be inserted into the cap as an internal part thereof, the insert having an inner surface defining a contact surface which is at least approximately adapted to the shape of the prepared or unprepared joint head, and the insert being selected from a group of inserts differing in wall thickness and/or shape for adapting the inner diameter and/or the inner shape of the cap to the diameter and/or the outer shape of the respective joint head, before inserting the insert.

49. Joint prosthesis according to claim 48 **characterized in that** the insert (86) has a centric opening and/or an eccentric opening (88) for receiving the cap-side telescopic member or the stem, respectively, and optionally an anti-twist member.

50. Joint prosthesis according to claims 40 and 48 or 49 **characterized in that** the insert and the sleeve extending over at least a part of the longitudinal extension of the telescopic arrangement or the stem, respectively, are firmly interconnected.

51. Joint prosthesis according to any one of the preceding claims **characterized in that** the cap includes an upper cap portion (89) which is rotatably mounted on a lower cap portion (90).

52. Joint prosthesis according to any one of the preceding claims **characterized in that** the cap (11') includes a flattened pole and/or subpole(80).

53. Joint prosthesis according to any one of the preceding claims **characterized by** a covering and/or cushioning of the supporting member at the side opposite to the bone contacting surface.

54. Joint prosthesis according to claim 8 or 9 **characterized in that** the hinge (19') is designed for being sunk in the drilling.

## Revendications

1. Prothèse articulaire pour le condyle, coopérant avec une glène, d'une articulation, en particulier d'une articulation de la hanche, comprenant :
- une calotte (11, 11', 11", 111) qui présente une surface d'application (13) pour appliquer la calotte au condyle et une surface extérieure (12) au moins approximativement de la forme d'une calotte sphérique,
- un ensemble télescopique (15) qui présente un élément télescopique côté calotte (17, 17", 17"', 18', 117, 117', 117") relié à la calotte (11, 11', 11", 111) et faisant saillie à partir de la calotte, et un élément télescopique côté pièce de retenue (17', 18, 18", 118) en engagement télescopique avec celui-ci, et qui est dimensionné et disposé de telle sorte qu'à l'état implanté, il s'étend à travers un perçage dans le condyle et des régions osseuses adjacentes à celui-ci, et
- une pièce de retenue (16) qui est en communication avec l'élément télescopique côté pièce de retenue (17', 18, 18", 118) et qui est réalisée pour être fixée sur une surface osseuse opposée au condyle,
l'élément télescopique côté calotte (17, 17", 17"', 18', 117, 117', 117") étant guidé de façon coulissante par rapport à l'élément télescopique côté pièce de retenue. (17', 18, 18", 118) dans la direction s'éloignant de la glène,
**caractérisée en ce que**
- la surface d'application est réalisée par une surface intérieure (13), adaptée au moins approximativement à la forme du condyle préparé ou non préparé de la calotte (11, 11', 11", 111), et
- un dispositif de blocage (30, 42, 67) agissant en fonction de la direction est prévu qui permet un réglage de la calotte par rapport à la pièce de retenue (16) dans la direction s'éloignant de la glène et l'empêche dans la direction orientée vers la glène ou le rend au moins plus difficile en comparaison avec un réglage dans la direction s'éloignant de la glène.

2. Prothèse articulaire selon la revendication 1, **caractérisée en ce que** l'ensemble télescopique (15) est réalisé de façon souple et/ou articulée et/ou l'ensemble télescopique est relié de façon souple et/ou articulée à la calotte (11, 11', 11", 111) et/ou à la pièce de retenue (16), de sorte qu'à l'état implanté, la prothèse articulaire permet au moins en partie les mouvements biomécaniques du condyle et du col par rapport à la tige de l'os concerné.

3. Prothèse articulaire selon la revendication 1 ou 2, **caractérisée en ce que** l'élément télescopique côté calotte est un élément enfichable (17, 17", 17"', 117, 117") et l'élément télescopique côté pièce de retenue est réalisé comme un élément de réception (18, 18", 118).

4. Prothèse articulaire selon la revendication 1 ou 2, **caractérisée en ce que** l'élément télescopique côté pièce de retenue est un élément enfichable (17') et l'élément télescopique côté calotte est réalisé comme un élément de réception (18').

5. Prothèse articulaire selon l'une des revendications précédentes, **caractérisée en ce que** la surface intérieure (13) de la calotte (11, 11', 11", 111) est réalisée au moins approximativement sous la forme d'une calotte sphérique.

6. Prothèse articulaire selon l'une des revendications précédentes, **caractérisée en ce que** comme pièce de retenue, une plaque de fixation (16) est prévue qui est reliée à l'extrémité détournée de la calotte (11, 11', 11", 111) de l'élément télescopique côté pièce de retenue (17', 18, 18", 118).

7. Prothèse articulaire selon la revendication 6, **caractérisée en ce que** l'élément télescopique côté pièce de retenue (17', 18, 18", 118) est relié de façon mobile à la plaque de fixation (16).

8. Prothèse articulaire selon la revendication 7, **caractérisée en ce que** l'élément télescopique côté pièce de retenue (17', 18, 18", 118) est articulé sur la plaque de fixation (16).

9. Prothèse articulaire selon la revendication 8, **caractérisée en ce que** l'élément télescopique côté pièce de retenue (17', 18, 18", 118) est relié à la plaque de fixation (16) par une articulation à charnière (19) dont l'axe de rotation est disposé substantiellement perpendiculairement à l'étendue longitudinale de l'ensemble télescopique (15) et substantiellement perpendiculairement à l'étendue longitudinale de la plaque de fixation.

10. Prothèse articulaire selon la revendication 8 ou 9, **caractérisée en ce que** l'élément télescopique côté pièce de retenue (118) est relié à la plaque de fixation (16) par une articulation à charnière (77) dont l'axe de rotation est disposé substantiellement perpendiculairement à l'étendue longitudinale de l'ensemble télescopique (15) et substantiellement en parallèle à l'étendue longitudinale de la plaque de fixation.

11. Prothèse articulaire selon l'une des revendications précédentes, **caractérisée en ce que** l'élément télescopique côté pièce de retenue (17', 18, 18", 118) s'appuie en faisant ressort sur la pièce de retenue (16).

12. Prothèse articulaire selon l'une des revendications 7 à 11, **caractérisée par** un élément de blocage pour le renforcement temporaire sélectif de la connexion mobile entre l'élément télescopique côté pièce de retenue (17', 18, 18", 118) et la plaque de fixation (16).

13. Prothèse articulaire selon les revendications 11 et 12, **caractérisée en ce que** pour l'appui faisant ressort de l'élément télescopique côté pièce de retenue (17', 18, 18", 118), un ressort à lames (22) s'appliquant contre l'élément télescopique côté pièce de retenue et la plaque de fixation (16), et **en ce que** l'élément de blocage est réalisé comme une plaque angulaire qui peut se substituer au ressort à lames si nécessaire.

14. Prothèse articulaire selon l'une des revendications précédentes, **caractérisée en ce que** l'ensemble télescopique (15) présente au moins un élément de flexion (40) permettant au moins en partie les mouvements biomécaniques du condyle.

15. Prothèse articulaire selon la revendication 14, **caractérisée en ce que** l'élément de flexion (40) est disposé à une certaine distance de la surface intérieure (13) de la calotte (11, 11', 11", 111) qui correspond à une à trois fois, en particulier à environ une fois et demi, le rayon intérieur de la calotte.

16. Prothèse articulaire selon la revendication 14 ou 15, **caractérisée en ce que** l'élément de flexion (40) est réalisé de façon résistante à la torsion.

17. Prothèse articulaire selon l'une des revendications 14 à 16, **caractérisée en ce que** l'élément de flexion (40) est réalisé comme un ressort résistant à la torsion, un arbre flexible, un arbre de torsion, une articulation à ressort, un insert ou similaires.

18. Prothèse articulaire selon l'une des revendications 14 à 16, **caractérisée en ce que** l'élément de flexion (40) est relié de façon intégrale à l'élément télescopique côté calotte (17, 17", 17"', 18', 117, 117').

19. Prothèse articulaire selon l'une des revendications précédentes, **caractérisée en ce qu'**au moins l'un des deux éléments télescopiques (17"') se compose de deux ou plusieurs pièces (60, 61), substantiellement situées successivement dans la direction longitudinale de l'ensemble télescopique, qui peuvent être emboîtées ou vissées ensemble de façon résistante à la rotation, et bloquées de façon résistante au détachement automatique.

20. Prothèse articulaire selon l'une des revendications précédentes, **caractérisée en ce que** le dispositif de blocage est réalisé comme un dispositif de couplage (30) rendu effectif de façon automatique en fonction de la direction, en particulier un dispositif d'arrêt ou d'encliquetage.

21. Prothèse articulaire selon la revendication 20, **caractérisée en ce que** le dispositif de couplage (30) présente au moins une protubérance d'arrêt (32, 63), faisant saillie sur l'un des éléments télescopiques (17, 17', 18", 117) ou l'une de leurs pièces (61), qui est formée et/ou chargée par une force de ressort de telle sorte qu'en cas de tentative de réglage de l'élément ou de la pièce télescopique côté calotte par rapport à l'élément ou à la pièce télescopique côté pièce de retenue dans la direction orientée vers la glène, il vient en engagement de blocage avec une surface d'application (37, 58) de l'autre élément ou pièce télescopique (17", 18, 18', 60) tout en permettant un réglage de l'élément ou de la pièce télescopique côté calotte par rapport à l'élément ou à la pièce télescopique côté pièce de retenue dans la direction s'éloignant de la glène.

22. Prothèse articulaire selon la revendication 21, **caractérisée en ce que** la protubérance d'arrêt (32, 63) est réalisée par au moins un ressort (31, 31', 55, 59) agissant radialement vers l'extérieur ou vers l'intérieur qui est fixé par rapport à l'un des éléments ou l'une des pièces télescopiques (17, 17', 17"', 18, 18", 117).

23. Prothèse articulaire selon la revendication 21, **caractérisée en ce que** la protubérance d'arrêt (32) est réalisée par au moins un ressort (55, 59) agissant radialement vers l'extérieur ou vers l'intérieur qui est relié de façon intégrale à l'un des éléments ou l'une des pièces télescopiques (117, 18").

24. Prothèse articulaire selon l'une des revendications 21 à 23, **caractérisée par** un anneau (47), pouvant être poussé au-delà de la protubérance d'arrêt (32), qui retient d'abord la protubérance d'arrêt et la libère seulement de façon automatique lorsque les deux éléments ou pièces télescopiques sont emboîté(e)s l'un(e) dans l'autre.

25. Prothèse articulaire selon l'une des revendications 21 à 24, **caractérisée en ce que** la surface d'application (37, 58) de l'autre élément ou pièce télescopique (17", 18, 18', 60) présente au moins un évidement d'arrêt (64), en particulier une pluralité d'évidements d'arrêt sous la forme d'un profil en dents de scie ou similaires.

26. Prothèse articulaire selon l'une des revendications 21 à 24, **caractérisée en ce que** la surface d'application de l'autre élément ou pièce télescopique est réalisée sans profil et/ou se compose d'un revêtement dans lequel la protubérance d'arrêt peut s'accrocher.

27. Prothèse articulaire selon l'une des revendications précédentes, **caractérisée par** un dispositif de précontrainte de ressort (42, 67) qui précontraint l'élément télescopique côté calotte (17, 117') dans la direction s'éloignant de la glène.

28. Prothèse articulaire selon la revendication 27, **caractérisée en ce que** le dispositif de précontrainte de ressort présente au moins un ressort de traction (42) qui est relié d'une part à l'élément télescopique côté calotte (17) et d'autre part à l'élément télescopique côté pièce de retenue (18) et/ou à la pièce de retenue (16).

29. Prothèse articulaire selon la revendication 27 ou 28, **caractérisée en ce que** le dispositif de précontrainte de ressort (67) forme en même temps le dispositif de blocage agissant en fonction de la direction.

30. Prothèse articulaire selon la revendication 29, **caractérisée en ce que** l'élément télescopique (117') réalisé comme un élément enfichable présente un épaulement extérieur (71) et l'élément télescopique (118) réalisé comme un élément de réception présente un épaulement intérieur et un ressort de précontrainte entre les deux épaulements.

31. Prothèse articulaire selon la revendication 30, **caractérisée en ce que** l'élément télescopique (117') réalisé comme un élément enfichable est formé par deux pièces (68, 69) disposées substantiellement de façon équiaxiale et reliées ensemble de façon amovible, en particulier vissées ensemble, dont l'une (68) est reliée à la calotte (11) et l'autre (69) porte l'épaulement extérieur (71).

32. Prothèse articulaire selon l'une des revendications précédentes, **caractérisée par** un ensemble (35, 35', 69, 270, 276) qui permet de désactiver le dispositif de blocage pour le démontage de la prothèse.

33. Prothèse articulaire selon l'une des revendications précédentes, **caractérisée en ce que** l'élément télescopique côté calotte (17, 17"', 18', 117, 117") s'éloigne substantiellement de façon centrée de la surface intérieure (13) de la calotte (11, 11', 11", 111).

34. Prothèse articulaire selon l'une des revendications précédentes, **caractérisée par** au moins un mécanisme antitorsion permettant d'empêcher toute rotation de la calotte (11, 11', 11", 111) par rapport au condyle.

35. Prothèse articulaire selon la revendication 34, **caractérisée en ce que** les deux éléments télescopiques (17, 18 ; 17', 18 '; 17", 18" ; 18, 117 ; 117', 118) se trouvent en connexion mutuelle résistante à la rotation.

36. Prothèse articulaire selon la revendication 35, **caractérisée en ce que** la section transversale des deux éléments télescopiques (17, 18 ; 17', 18' ; 17", 18" ; 18, 117 ; 117', 118) est profilée de telle sorte que la connexion mutuelle résistante à la rotation est assurée.

37. Prothèse articulaire selon l'une des revendications 34 à 36, **caractérisée en ce que** l'élément télescopique côté calotte (17) est disposé de façon excentrique à une distance prédéterminée par rapport à l'axe médian ou à la périphérie de la calotte (11).

38. Prothèse articulaire pour la tête de fémur, coopérant avec une glène, d'une articulation de la hanche, comprenant :
- une calotte (11) qui présente une surface d'application (13) pour appliquer la calotte au condyle et au moins une surface extérieure approximativement de la forme d'une calotte sphérique, et
- au moins une pièce de retenue (16) réalisée pour être fixée à une surface osseuse opposée au condyle,
**caractérisée en ce que**
- la surface d'application est réalisée par une surface intérieure (13), adaptée au moins approximativement à la forme du condyle préparé ou non préparé de la calotte (11), et
- au moins un dispositif de blocage (93) est prévu qui rend plus difficile un réglage mutuel entre la calotte et la pièce de retenue (16) dans la direction orientée vers la glène en comparaison avec un réglage dans la direction s'éloignant de la glène, le dispositif de blocage présentant au moins un élément de traction (94), en particulier faisant ressort, qui, à l'état implanté, s'étend à travers un perçage dans le condyle et dans des parties osseuses adjacentes à celui-ci, et l'élément de traction étant relié d'une part à la calotte et d'autre part à la pièce de retenue et précontraint la calotte dans la direction s'éloignant de la glène.

39. Prothèse articulaire selon la revendication 38, **caractérisée en ce qu'**au moins une tige (96) s'éloigne de la surface intérieure de la calotte (11) et l'élément de traction (94) s'engage à l'extrémité de la tige éloignée de la surface intérieure de calotte (13).

40. Prothèse articulaire selon l'une des revendications précédentes, **caractérisée par** un manchon qui peut être inséré dans le perçage et qui, à l'état implanté, entoure l'ensemble télescopique ou l'élément de traction et/ou la tige sur au moins une partie de sa dimension longitudinale.

41. Prothèse articulaire selon l'une des revendications 38 à 40, **caractérisée par** au moins un mécanisme antitorsion (50 ; 100, 101) permettant d'empêcher toute rotation de la calotte (11) par rapport à la glène.

42. Prothèse articulaire selon l'une des revendications 34 à 41, **caractérisée en ce que** la calotte (11) porte sur sa surface intérieure au moins un élément antitorsion (50 ; 100, 101) permettant d'empêcher toute rotation de la calotte par rapport au condyle.

43. Prothèse articulaire selon l'une des revendications 34 à 42, **caractérisée en ce que** la calotte est munie d'au moins un perçage (100) disposé de façon excentrique dans lequel un élément antitorsion (101) donné peut être entré et encastré jusqu'à pénétration dans le condyle.

44. Prothèse articulaire selon l'une des revendications 39 à 43, **caractérisée en ce que** la tige (96, 102) est disposée de façon centrée ou de façon excentrique à une distance prédéterminée de l'axe médian ou de la périphérie de la calotte (11).

45. Prothèse articulaire selon l'une des revendications précédentes, **caractérisée en ce que** l'ensemble télescopique ou la tige présente au moins au niveau de la calotte du côté extérieur une section transversale circulaire.

46. Prothèse articulaire selon l'une des revendications précédentes, **caractérisée en ce que** le bord (81) de la calotte (11") se trouve au moins pour la plus grande partie dans un plan qui est situé sous un angle incliné par rapport à l'axe médian de la prothèse, de telle sorte que le condyle est recouvert par la calotte à l'état implanté au moins approximativement de façon homogène.

47. Prothèse articulaire selon l'une des revendications précédentes, **caractérisée en ce que** la calotte (111) est munie d'évidements (84) qui sont de préférence ébavurés sur la surface extérieure ou réalisés en forme d'entonnoir.

48. Prothèse articulaire selon l'une des revendications précédentes, **caractérisée par** au moins un insert (86) pouvant être inséré dans la calotte comme une partie intérieure de la calotte, la face intérieure de l'insert comme surface d'application étant au moins approximativement adaptée à la forme du condyle préparé ou non préparé, et l'insert étant choisi et mis en oeuvre parmi un groupe d'inserts avec différentes épaisseurs de paroi et/ou différentes formes permettant d'adapter le diamètre intérieur et/ou la forme intérieure de la calotte au diamètre et/ou à la forme extérieure du condyle respectif.

49. Prothèse articulaire selon la revendication 48, **caractérisée en ce que** l'insert (86) est muni d'un évidement centré et/ou excentrique (88) permettant de faire passer l'élément télescopique côté calotte ou la tige et le cas échéant un élément antitorsion.

50. Prothèse articulaire selon les revendications 40 et 48 ou 49, **caractérisée en ce que** l'insert et le manchon s'étendant sur au moins une partie de la dimension longitudinale de l'ensemble télescopique ou de la tige sont reliés solidement ensemble.

51. Prothèse articulaire selon l'une des revendications précédentes, **caractérisée en ce qu'**une partie de la calotte est un dessus de calotte (89) qui est logé de façon rotative sur un dessous de calotte (90).

52. Prothèse articulaire selon l'une des revendications précédentes, **caractérisée en ce que** le pôle et/ou le pôle inférieur (80) de la calotte (11') est aplati.

53. Prothèse articulaire selon l'une des revendications précédentes, **caractérisée par** un recouvrement et/ou un rembourrage de la pièce de retenue du côté détourné de la surface d'application de l'os.

54. Prothèse articulaire selon la revendication 8 ou 9, **caractérisée en ce que** l'articulation (19') est réalisée pour une disposition encastrée dans le perçage.
